# FASCICULE DE BREVET EUROPEEN

(11) **EP 1 252 150 B1**
(45) Date de publication et mention de la délivrance du brevet: **04.06.2003**
(21) Numéro de dépôt: 01904021.1
(22) Date de dépôt: 01.02.2001
(51) Int. Cl.: C07D 263/58, C07D 277/68, C07D 417/06, C07D 498/04, C07D 471/04, A61K 31/423, A61K 31/428, A61K 31/4353, A61P 3/10, A61P 3/06

(54) **DERIVES D'AZOLES CONDENSES ET LEUR UTILISATION COMME AGENTS HYPOGLYCEMIANTS**
KONDENSIERTE AZOLDERIVATE UND IHRE VERWENDUNG ZUR SENKUNG DES BLUTZUCKERSPIEGELS
HETEROCYCLIC DERIVATIVES, PREPARATION METHOD AND PHARMACEUTICAL COMPOSITIONS CONTAINING SAME

(30) Priorité: 02.02.2000 FR 0001289
(43) Date de publication de la demande: 30.10.2002
(73) Titulaire: Les Laboratoires Servier, 92415 Courbevoie Cedex (FR)
(72) Inventeur: LESIEUR, Daniel, 59147 Gondecourt (FR); BLANC-DELMAS, Elodie, 59000 Lille (FR); YOUS, Said, 59120 Loos (FR); DEPREUX, Patrick, 59280 Armentières (FR); GUILLAUMET, Gérald, 45650 Saint Jean le Blanc (FR); DACQUET, Catherine, 75017 Paris (FR); LEVENS, Nigel, 92420 Vaucresson (FR); BOUTIN, Jean Albert, 92150 Suresnes (FR); BENNEJEAN, Caroline, 94220 Charenton Le Pont (FR); RENARD, Pierre, 78150 Le Chesnay (FR)
(86) Numéro de dépôt international: FR0100304
(87) Numéro de publication internationale: WO01057002

(56) Documents cités:
- EP-A- 0 513 580
- EP-A- 0 779 279
- CRAMER R D ET AL: "Prospective identification of biologically active structures by topomer shape similarity searching" JOURNAL OF MEDICINAL CHEMISTRY,AMERICAN CHEMICAL SOCIETY. WASHINGTON,US, vol. 42, no. 19, 1999, pages 3919-3933, XP002149047 ISSN: 0022-2623
- LOHRAY B B ET AL: "Novel euglycemic and hypolipidemic agents. 1" JOURNAL OF MEDICINAL CHEMISTRY,AMERICAN CHEMICAL SOCIETY,US, vol. 41, no. 10, 1998, pages 1619-1630, XP002149048 ISSN: 0022-2623

## Description

La présente invention concerne de nouveaux dérivés hétérocycliques, leur procédé de préparation et les compositions pharmaceutiques qui les contiennent.

Les composés décrits dans la présente invention sont nouveaux et présentent des propriétés pharmacologiques particulièrement intéressantes : ce sont d'excellents agents hypoglycémiants et hypolipémiants.

Le traitement du diabète non insulino-dépendant de type II reste non satisfaisant en dépit de la mise sur le marché de nombreux dérivés hypoglycémiants oraux destinés à faciliter la sécrétion d'insuline et à favoriser son action au niveau des tissus cibles périphériques.

Lors des dix dernières années, une classe de composés de structure thiazolidinediones (US 5089514, US 5306726) a montré une activité antidiabétique marquée en favorisant la sensibilité à l'insuline dans les tissus périphériques cibles (muscles squelettiques, foie, tissu adipeux) de modèles animaux de diabète non insulino-dépendant de type II. Ces composés réduisent également les taux d'insuline et de lipides dans ces mêmes modèles animaux et induisent *in vitro* la différenciation de lignées cellulaires de préadipocytes en adipocytes (A. Hiragun et al., J. Cell. Physiol., 1988, 134, 124-130 ; R.F. Kleitzen et al., Mol. Pharmacol., 1992, 41, 393-398).

Le traitement des lignées cellulaires préadipocytaires avec la thiazolidinedione Rosiglitazone entraîne une induction de l'expression de gènes spécifiques du métabolisme lipidique comme aP2 et l'adipsine ainsi que l'expression des transporteurs du glucose GLUT1 et GLUT4, suggérant que l'effet des thiazolidinediones observé *in vivo* peut-être médié via le tissu adipeux. Cet effet spécifique est obtenu par la stimulation des facteurs nucléaires de transcription : « peroxisome proliferator-activated receptor gamma » (PPAR γ2). Ces dérivés sont susceptibles de restaurer la sensibilité à l'insuline au niveau des tissus périphériques tels que le tissu adipeux ou les muscles squelettiques (J.E. Gerich, New Engl. Med., 19, 321, 1231-1245).

Néanmoins, les dérivés de structure thiazolidinediones (troglitazone, rosiglitazone) ont montré chez l'homme des effets secondaires inquiétants, notamment des problèmes hépatiques (Script N° 2470, 1999, Sept. 8^{th}, 25).

Certains dérivés de structure [[(hétérocyclyl)èthoxy]benzyl]-2,4-thiazolidinedione, dans laquelle le reste hétérocyclyl peut être un reste 1-indolyl, 1-indolenyl ou 1-pyrrolo[2,3-*b*]pyridinyl, présentent une activité euglycémiante et hypolipémiante (B. B. Lohray et al., J. Med. Chem., 1998, 41, 1619-1630).

De nombreux agents hypoglycémiants présentent des effets secondaires importants (hépatiques, cardiaques, hématopoïétiques) qui limitent leur utilisation à long terme dans le traitement du diabète non insulino-dépendant de type II.

Le développement de nouveaux agents thérapeutiques moins toxiques et actifs à long terme est absolument nécessaire dans cette pathologie.

Par ailleurs, l'hyperlipidémie est souvent observée chez les diabétiques (Diabète Care, 1995, 18 (supplément 1), 86/8/93). L'association de l'hyperglycémie avec l'hyperlipidémie favorise le risque de maladies cardiovasculaires chez les diabétiques. L'hyperglycémie, l'hyperlipidémie et l'obésité sont devenues des pathologies du monde moderne marqué par une prise de nourriture en grande quantité et un manque chronique d'exercice.

L'augmentation de la fréquence de ces pathologies appelle au développement de nouveaux agents thérapeutiques actifs dans ces maladies : des composés présentant une excellente activité hypoglycémiante et hypolipémiante en évitant les effets secondaires observés avec les thiazolidinediones sont par conséquent très utiles dans le traitement et/ou la prophylaxie de ces pathologies et particulièrement indiqués dans le traitement des diabètes non insulino-dépendants de type II pour réduire l'insulino-résistance périphérique et normaliser le contrôle du glucose.

Les composés de la présente invention, outre leur nouveauté, répondent à ces critères pharmacologiques et constituent d'excellents agents hypoglycémiants et hypolipémiants.

La présente invention concerne plus particulièrement les composés de formule (I): 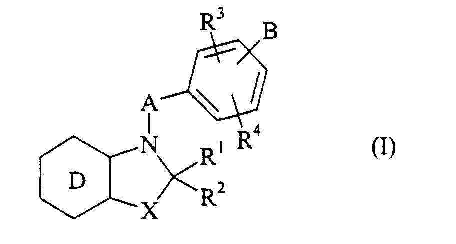 dans laquelle :
- X représente un atome d'oxygène ou de soufre, ou un groupement CH₂ ou 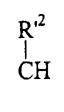 (dans lequel R'² forme avec R² une liaison supplémentaire),
- R¹ et R², identiques ou différents représentent un atome d'hydrogène, un groupement alkyle (C₁-C₆) linéaire ou ramifié, aryle, arylalkyle (C₁-C₆) linéaire ou ramifié, aryloxy, arylalkyloxy (C₁-C₆) linéaire ou ramifié, alkoxy (C₁-C₆) linéaire ou ramifié, hydroxy, amino, alkylamino (C₁-C₆) linéaire ou ramifié ou dialkylamino (C₁-C₆) linéaire ou ramifié,
   ou R¹ et R² forment ensemble un groupement oxo, thioxo ou imino,
   R² pouvant de plus former avec R'² une liaison supplémentaire,
- A représente une chaîne alkylène (C₁-C₆) dont un groupement CH₂ peut être remplacé par un hétéroatome choisi parmi oxygène ou soufre, ou par un groupement NRₐ (où Rₐ représente un atome d'hydrogène ou un groupement alkyle (C₁-C₆) linéaire ou ramifié), ou par un groupement phénylène ou naphtylène,
- B représente un groupement alkyle (C₁-C₆) linéaire ou ramifié, ou alkényle (C₂-C₆) linéaire ou ramifié, ces groupements étant substitués par un groupement R⁵, par un groupement de formule (II) 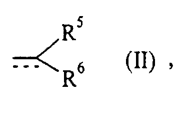 (II) , ou par un groupement de formule (III) 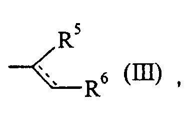 dans lesquels :
   - la représentation ---- signifie que la liaison est simple ou double,
   - R⁵ représente un groupement 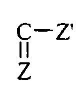
   dans lequel Z représente un atome de soufre ou d'oxygène et Z' représente un groupement OR ou NRR',
   - et R⁶ représente un groupement 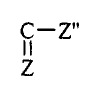
   dans lequel Z" représente un groupement Z' ou R,
   (où R et R', identiques ou différents, représentent un groupement R" ou ―C(Me)₂COOR" où R" représente un atome d'hydrogène ou un groupement alkyle (C₁-C₆) linéaire ou ramifié, alkényle (C₂-C₆) linéaire ou ramifié, alkynyle (C₂-C₆) linéaire ou ramifié, aryle, arylalkyle (C₁-C₆) linéaire ou ramifié, arylalkényle (C₂-C₆) linéaire ou ramifié, arylalkynyle (C₂-C₆) linéaire ou ramifié, hétéroaryle, hétéroarylalkyle (C₁-C₆) linéaire ou ramifié, hétéroarylalkényle (C₂-C₆) linéaire ou ramifié, hétéroarylalkynyle (C₂-C₆) linéaire ou ramifié, cycloalkyle (C₃-C₈), cycloalkyl(C₃-C₈)alkyle(C₁-C₆) linéaire ou ramifié ou polyhalogénoalkyle (C₁-C₆) linéaire ou ramifié),
- R³ et R⁴, identiques ou différents, représentent un atome d'hydrogène ou d'halogène, ou un groupement R, OR ou NRR' (où R et R' sont tels que définis précédemment), ou R³ et R⁴ forment ensemble avec les atomes de carbone qui les portent, lorsqu'ils sont portés par deux atomes de carbone adjacents, un cycle comportant 5 ou 6 chaînons et pouvant contenir un hétéroatome choisi parmi oxygène, soufre et azote,
- D représente :
   un noyau benzénique et dans ce cas X ne peut représenter un groupement 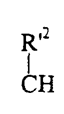 tel que défini précédemment,
   ou D représente un noyau pyridinique, pyrazinique, pyrimidinique ou pyridazinique, ces noyaux étant non substitués ou substitués par 1 à 3 groupements, identiques ou différents, choisis parmi R, OR, S(O)ₙR, C(Z)R 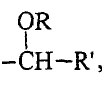 C(Z)OR, NRR', C(Z)NRR', 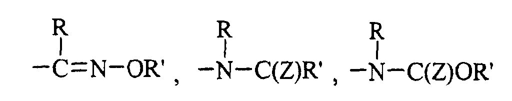 (où R, R' et Z sont tels que définis précédemment et n vaut 0, 1 ou 2), cyano, nitro ou atomes d'halogène,
étant entendu que :
* lorsque A représente un groupement CH₂, B ne peut représenter un groupement alkyle (C₁-C₆) linéaire ou ramifié substitué par un groupement 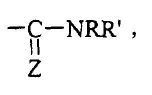
* lorsque les groupements A et B sont en ortho l'un par rapport à l'autre sur le noyau benzénique qui les porte, B ne peut représenter un groupement alkénylène (C₂-C₆) linéaire ou ramifié substitué par un groupement 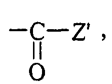
* lorsque A représente un groupement 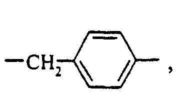 B ne peut représenter un groupement ―CH₂-COOH,
* par aryle on entend un groupement phényle, naphtyle ou biphényle, ces groupements pouvant être partiellement hydrogénés,
* par hétéroaryle on entend tout groupement aromatique mono ou bicyclique contenant 5 à 10 chaînons, pouvant être partiellement hydrogéné sur un des cycles dans le cas des hétéroaryles bicycliques, et contenant 1 à 3 hétéroatomes choisis parmi oxygène, azote et soufre,
les groupements aryle et hétéroaryle ainsi définis pouvant être substitués par 1 à 3 groupements choisis parmi alkyle (C₁-C₆) linéaire ou ramifié, alkoxy (C₁-C₆) linéaire ou ramifié, carboxy, formyle, NR_{b}R_{c} (dans lequel R_{b} et R_{c}, identiques ou différents, représentent un atome d'hydrogène, un groupement alkyle (C₁-C₆) linéaire ou ramifié, aryle ou hétéroaryle), ester, amido, nitro, cyano, O-C(Me)₂COOR" (où R" est tel que défini précédemment), ou atomes d'halogène,
leurs énantiomères et diastéréoisomères ainsi que leurs sels d'addition à un acide ou à une base pharmaceutiquement acceptables.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, méthanesulfonique, camphorique, oxalique, etc...

Parmi les bases pharmaceutiquement acceptables, on peut citer à titre non limitatif l'hydroxyde de sodium, l'hydroxyde de potassium, la triéthylamine, la tertbutylamine, etc...

Les composés préférés de l'invention sont les composés de formule (I) pour lesquels R¹ et R² forment ensemble un groupement oxo.

Le groupement préféré pour R³ et R⁴ est l'atome d'hydrogène.

Préférentiellement, A représente une chaîne alkylène dont un groupement CH₂ peut être remplacé par un hétéroatome.

Plus particulièrement, l'invention concerne les composés de formule (I) pour lesquels A représente un groupement éthylèneoxy.

Les groupements D préférés sont le noyau benzénique et le noyau pyridinique.

Les groupements D préférés sont le noyau benzénique et le noyau pyridinique, ces groupements étant non substitués ou substitués, préférentiellement en position 6, et plus particulièrement par un groupement C(Z)R (comme les groupements benzoyle, halobenzoyle, biphénylcarbonyle, naphtoyle, alkoyle, pyridinylcarbonyle, alkoxybenzoyle, alkylbenzoyle), R (comme par exemple les groupements benzyle, halobenzyle, alkylbenzyle, alkoxybenzyle, biphénylméthyle, phénylvinyle), 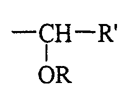 (comme par exemple (hydroxy)(phényl)méthyl), 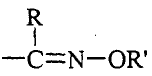 (comme par exemple (alkoxyimino) (phényl)méthyle, (hydroxyimino)(phényl)méthyle, (alkoxyimino)(halophényl)méthyle), C(Z)NRR'(comme par exemple le groupement CONHPh), ou 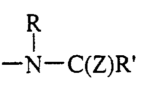 (comme par exemple le groupement NHCOPh).

X représente préférentiellement un atome d'oxygène ou de soufre ou lorsque D représente un noyau pyridinique un groupement CHR'².

Les groupements B préférés sont :
- les groupements alkyle ou alkényle substitués par un groupement de formule (II) et plus particulièrement les groupements alkyle substitués par un groupement de formule (II) comme par exemple : - le groupement 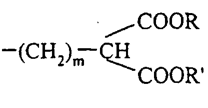 dans lequel m vaut 1 ou 2 et R et R', identiques ou différents, représentent un atome d'hydrogène ou un groupement alkyle comme méthyle ou éthyle par exemple
   - le groupement 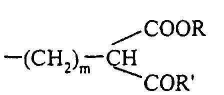 dans lequel m vaut 1 ou 2, R représente préférentiellement un atome d'hydrogène ou un groupementalkyle comme méthyle ou éthyle par exemple, et R' représente avantageusement un groupement aryle comme par exemple phényle éventuellement substitué,
   - ou le groupement 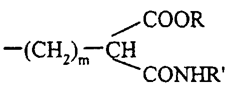 dans lequel m vaut 1 ou 2 et R et R', identiques ou différents, représentent un atome d'hydrogène ou un groupement alkyle comme méthyle ou éthyle par exemple,
- les groupements alkyle ou alkényle substitués par un groupement R⁵ et plus particulièrement les groupements alkyle substitués par un groupement R⁵ comme par exemple le groupement -(CH₂)ₚ-COOR dans lequel p vaut 1, 2 ou 3 et R représente un atome d'hydrogène ou un groupement alkyle comme méthyle ou éthyle par exemple ou aryle comme phényle par exemple.

Encore plus particulièrement, l'invention concerne les composés de formule (I) qui sont :
* le 2-{4-[2-(2-oxo-1,3-benzoxazol-3(2*H*)-yl)éthoxy]benzylidène}malonate de diéthyle,
* le 2-{4-[2-(2-oxo-1,3-benzoxazol-3(2*H*)-yl)éthoxy]benzyl}malonate de diéthyle,
* l'acide 3-éthoxy-3-oxo-2- {4-[2-(2-oxo-1,3-benzoxazol-3(2*H*)-yl)éthoxy]benzyl} propanoïque,
* l'acide 2-{4-[2-(2-oxo-1,3-benzoxazol-3(2*H*)-yl)éthoxy]benzyl}malonique,
* le 2-{4-[2-(2-oxo-1,3-benzothiazol-3(2*H*)-yl)éthoxy]benzylidène}malonate de diéthyle,
* le 2-{4-[2-(2-oxo-1,3-benzothiazol-3(2*H*)-yl)éthoxy]benzylidène}malonate de diméthyle,
* le 2-{4-[2-(2-oxo-1,3-benzothiazol-3(2*H*)-yl)éthoxy]benzyl}malonate de diéthyle,
* le 2-{4-[2-(2-oxo-1,3-benzothiazol-3(2*H*)-yl)éthoxy]benzyl}malonate de diméthyle,
* l'acide 3-méthoxy-3-oxo-2-{4-[2-(2-oxo-1,3-benzothiazol-3(2*H*)-yl)éthoxy]benzyl} propanoïque,
* l'acide 3-éthoxy-3-oxo-2-{4-[2-(2-oxo-1,3-benzothiazol-3(2*H*)-yl)éthoxy]benzyl}propanoïque,
* l'acide 2-{4-[2-(2-oxo-1,3-benzothiazol-3(2*H*)-yl)éthoxy]benzyl}malonique,
* le 2-(4-[3-(2-oxo-1,3-benzothiazol-3(2*H*)-yl)propoxy]benzylidène}malonate de diéthyle,
* le 2-{4-[2-(2-oxo-1,3-benzothiazol-3(2*H*)-yl)éthoxy]benzylidène}propanedioate de *tert*-butyle et de méthyle,
* le 2-{4-[2-(2-oxo-1,3-benzothiazol-3(2*H*)-yl)éthoxy]benzyl}malonate de *tert*-butyle et de méthyle,
* le 2-{4-[2-(6-benzoyl-2-oxo-1,3-benzothiazol-3(2*H*)-yl)éthoxy]benzylidène}malonate de diméthyle,
* le 2-(4-[2-(6-benzoyl-2-oxo-1,3-benzothiazol-3(2*H*)-yl)éthoxy]benzyl}malonate de diméthyle,
* le 2-{4-[2-(6-benzyl-2-oxo-1,3-benzothiazol-3(2*H*)-yl)éthoxy]benzyl}malonate de diméthyle,
* le 2-{4-[2-(6-benzoyl-2-oxo-1,3-benzoxazol-3(2*H*)-yl)éthoxy]benzylidène}malonate de diméthyle,
* le 2-{4-[2-(6-benzoyl-2-oxo-1,3-benzoxazol-3(2*H*)-yl)éthoxy]benzyl}malonate de diméthyle,
* le 2-{4-[2-(6-benzyl-2-oxo-1,3-benzoxazol-3(2*H*)-yl)éthoxy]benzyl}malonate de diméthyle,
* l'acide 3-{4-[2-(2-oxo-1,3-benzoxazol-3(2*H*)-yl)éthoxy]phényl}-2-propénoïque,
* l'acide 3-{4-[2-(2-oxo-1,3-benzoxazol-3(2*H*)-yl)éthoxy]phényl}-2-propanoïque,
* l'acide 3-{4-[2-(2-oxo-1,3-benzothiazol-3(2*H*)-yl)éthoxy]phényl}-2-propénoïque,
* l'acide 3-{4-[2-(2-oxo-1,3-benzothiazol-3(2*H*)-yl)éthoxy]phényl}-2-propanoïque,
* le 2-{4-[2-(2-oxo[1,3]oxazolo[4,5-*b*]pyridin-3(2*H*)-yl)éthoxy]benzyl}malonate de diméthyle,
* le 2-{4-[2-(1*H*-pyrrolo[2,3-*b*]pyridin-1-yl)éthoxy]benzyl}malonate de diméthyle,
* le 3-{4-[2-(6-benzoyl-2-oxo-1,3-benzothiazol-3(2*H*)-yl)éthoxy]phényl}propanoate de méthyle,
* le 2-{4-[2-(6-[hydroxy(phényl)méthyl]-2-oxo-1,3-benzoxazol-3(2*H*)-yl)éthoxy] benzyl}malonate de diméthyle,
* le 3-{4-[2-(6-benzoyl-2-oxo-1,3-benzoxazol-3(2*H*)-yl)éthoxy]phényl}propanoate de méthyle,
* le 2-{4-[2-(6-[hydroxy(phényl)méthyl]-2-oxo-1,3-benzothiazol-3(2*H*)-yl) éthoxy] benzyl}malonate de diméthyle,
* le 2-{4-[2-(6-[(méthoxyimino)(phényl)méthyl]-2-oxo-1,3-benzothiazol-3(2*H*)-yl) éthoxy]benzyl}malonate de diméthyle,
* le 2-{4-[2-(6-(2-chlorobenzoyl)-2-oxo-1,3-benzothiazol-3(2*H*)-yl)éthoxy]benzyl} malonate de diméthyle,
* le 2-{4-[2-(6-[(hydroxyimino)(phényl)méthyl]-2-oxo-1,3-benzothiazol-3(2*H*)-yl) éthoxy]benzyl}malonate de diméthyle,
* le 2-{4-[2-(6-([1,1'-biphényl]-4-ylcarbonyl)-2-oxo-1,3-benzothiazol-3(2*H*)-yl)éthoxy] benzyl}malonate de diméthyle,
* le 2-{4-[2-(6-([1,1'-biphényl]4-ylméthyl)-2-oxo- 1,3-benzothiazol-3(2*H*)-yl)éthoxy] benzyl}malonate de diméthyle,
* le 2-{4-[2-(6-(1-naphtoyl)-2-oxo-1,3-benzothiazol-3(2*H*)-yl)éthoxy]benzyl}malonate de diméthyle,
* le 2-{4-[2-(6-(3-chlorobenzoyl)-2-oxo 1,3-benzothiazo]-3(2*H*)-yl) éthoxy]benzyl} malonate de diméthyle,
* le 2- {4-[2-(6-(2-chlorobenzyl)-2-oxo-1,3-benzothiazol-3(2*H*)-yl)éthoxy] benzyl} malonate de diméthyle,
* le 2-{4-[2-(6-(3-chlorobenzyl)-2-oxo-1,3-benzothiazol-3(2*H*)-yl)ethoxy] benzyl} malonate de diméthyle,
* le 2-{4-[2-(6-(1-naphtylméthyl)-2-oxo-1,3-benzothiazol-3(2*H*)-yl) éthoxy]benzyl} malonate de diméthyle,
* le 2-{4-[2-(2-oxo-6-(3-pyridinylcarbonyl)-1,3-benzothiazol-3(2*H*)-yl) éthoxy]benzyl} malonate de diméthyle,
* le 2-{4-[2-(6-(4-chlorobenzoyl)-2-oxo-1,3-berlzothiazol-3(2*H*)-yl) éthoxy]benzyl} malonate de diméthyle,
* l'acide 2-{4-[2-(6-benzoyl-2-oxo-1,3-benzothiazol-3(2*H*)-yl)éthoxy]benzyl} malonique,
* le 2-{4-[2-(6-(2-naphtoyl)-2-oxo-1,3-benzothiazol-3(2*H*)-yl)éthoxy]benzyl}malonate de diméthyle,
* le 2-(4-[2-(6-(4-méthoxybenzoyl)-2-oxo-1,3-benzothiazol-3(2*H*)-yl)éthoxy]benzyl} malonate de diméthyle,
* le 2-{4-[2-(6-(4-chlorobenzyl)-2-oxo-1,3-benzothiazol-3(2*H*)-yl) éthoxy]benzyl} malonate de diméthyle,
* l'acide 2-{4-[2-(6-benzoyl-2-oxo-1,3-benzothiazol-3(2*H*)-yl)étboxy]benzyl}-3-méthoxy-3-oxopropanoique,
* le 2-{4-[2-(6-[(3-chlorophényl)(méthoxyimino)méthyl]-2-oxo-1,3-benzothiazol-3 (2*H*)-yl)éthoxy]benzyl}malonate de diméthyle,
* le 2-{4-[2-(6-[[1,1'-biphényl]-4-yl(méthoxyimino)méthyl]-2-oxo-1,3-benzothiazol-3(2*H*)-yl)éthoxy]benzyl}malonate de diméthyle,
* l'acide 3-méthoxy-3-oxo-2-{4-[2-(1*H*-pyrrolo[2,3-*b*]pyridin-1-yl) éthoxy]benzyl} propanoique,
* le 2-{4-[2-(6-(benzoylamino)-2-oxo 1,3-benzothiazol-3 (2H)-yl)éthoxy] benzyl} malonate de diméthyle,
* le 3-{4-[2-(6-benzyl-2-oxo-1,3-benzothiazol-3(2*H*)-yl)éthoxy]phényl}propanoate de méthyle,
* le 3-{4-[2-(6-[(méthoxyimino)(phényl)méthyl]-2-oxo-1,3-benzothiazol-3(2*H*)-yl) éthoxy]phényl}propanoate de méthyle,
* l'acide 3-{4-[2-(6-benzoyl-2-oxo-1,3-benzothiazol-3(2*H*)-yl)éthoxy]phényl}propanoique,
* l'acide 2-{4-[2-(1*H*-pyrrolo[2,3-*b*]pyridin-1-yl)éthoxy]benzyl}malonique,
* le 2-{4-[2-(6-benzoyl-2-oxo-1,3-benzothiazol-3(2*H*)-yl)éthoxy]benzyl)-3-(méthylamino)-3-oxopropanoate de méthyle,
* le 2-{4-[2-(6-(anilinocarbonyl)-2-oxo-1,3-benzothiazol-3(2*H*)-yl)éthoxy]benzyl} malonate de diméthyle,
* le 2-(4-{[2-(6-benzoyl-2-oxo-1,3-benzothiazol-3(2*H*)-yl)éthyl]amino}benzyl)malonate de diméthyle,
* le 2-{4-[2-(2-oxo-6-[2-phényléthényl]-1,3-benzothiazol-3(2*H*)-yl)éthoxy]benzyl} malonate de diméthyle.

Les énantiomères, diastéréoisomères ainsi que les sels d'addition à un acide ou à une base pharmaceutiquement acceptables des composés préférés de l'invention font partie intégrante de l'invention.

La présente invention concerne également le procédé de préparation des composés de formule (I) caractérisé en ce que l'on utilise comme produit de départ un composé de formule (IV) : 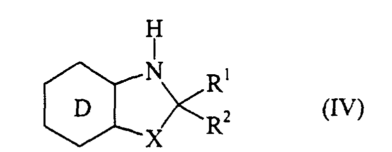 dans laquelle R¹, R², X et D sont tels que définis dans la formule (I),
sur lequel on condense en milieu basique un composé de formule (V) : 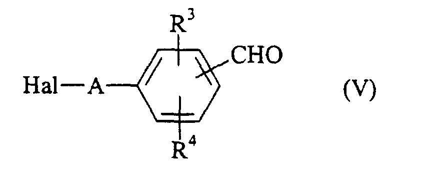 dans laquelle Hal représente un atome d'halogène et A, R³ et R⁴ sont tels que définis dans la formule (I),
pour conduire au composé de formule (VI) : 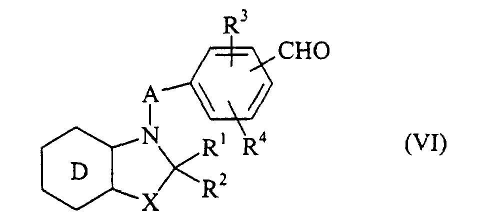 dans laquelle A, D, X, R¹, R², R³ et R⁴ sont définis de la même façon que précédemment,
- sur lequel on condense en milieu basique un composé de formule (VII) : 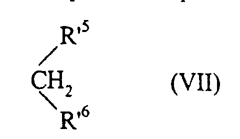
dans laquelle R'⁵ et R'⁶ peuvent prendre toutes les valeurs définies précédemment pour R⁵ et R⁶ à l'exception du groupement COOH,
pour obtenir le composé de formule (I/a), cas particulier des composés de formule (I) : 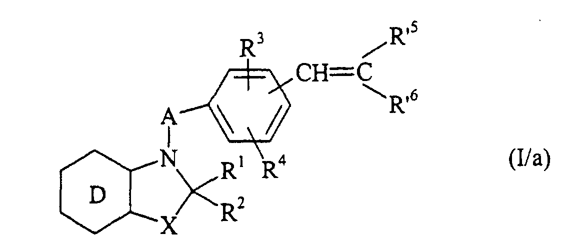 dans laquelle D, A, X, R¹, R², R³, R⁴, R'⁵ et R'⁶ sont définis comme précédemment,
qui est hydrogéné en présence d'un catalyseur comme le palladium sur charbon par exemple pour conduire au composé de formule (I/b), cas particulier des composés de formule (I): 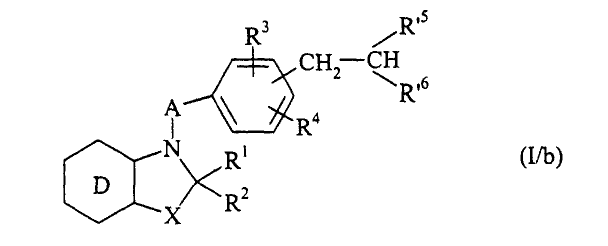 dans laquelle D, A, X, R¹, R²_{,} R³, R⁴, R'⁵ et R'⁶ sont tels que définis précédemment,
(les composés de formule (I/a) et (I/b) pouvant être obtenus par condensation directe sur le composé de formule (IV) d'un composé de formule (V') : 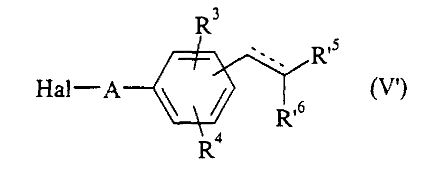 dans laquelle Hal représente un atome d'halogène et A, R³, R⁴, R'⁵ et R'⁶ sont tels que définis précédemment),
- ou composé de formule (VI) sur lequel on condense, dans les conditions d'une réaction de Horner-Emmons le composé phosphonique correspondant d'un dérivé de formule (VIII):

   Hal-CH₂-B' (VIII)

   dans laquelle Hal représente un atome d'halogène et B' représente un groupement alkyle (C₁-C₅) linéaire ou ramifié ou alkényle (C₂-C₅) linéaire ou ramifié, ces groupements étant substitués par un groupement R'⁵ ou par un groupement de formule (II') 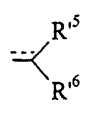 ou par un groupement de formule (III') 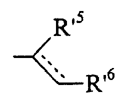 dans lesquels R'⁵ et R'⁶ et la représentation ---- sont tels que définis précédemment,
   pour conduire au composé de formule (I/c), cas particulier des composés de formule (I): 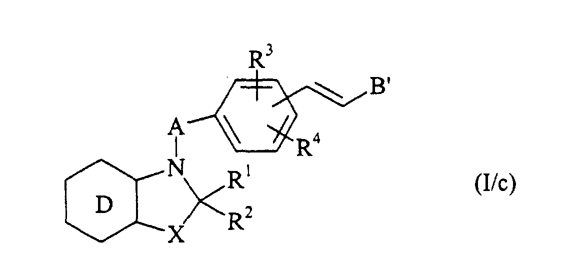 dans laquelle D, X, A, B', R¹, R², R³ et R⁴ sont tels que définis précédemment,
   qui peut être soumis à une hydrogénation catalytique, en présence de palladium par exemple pour obtenir le composé de formule (I/d), cas particulier des composés de formule (I): 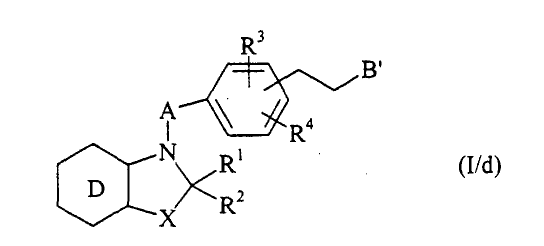 dans laquelle D, A, X, B', R¹, R², R³ et R⁴ sont tels que définis précédemment,
   - ou composé de formule (VI) que l'on transforme en chlorure d'acide correspondant sur lequel on condense, en présence d'un dérivé du palladium ou de l'étain par exemple, le composé de formule (IX) : 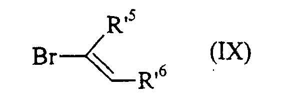
   dans laquelle R'⁵ et R'⁶ sont tels que définis précédemment,
   pour conduire au composé de formule (X) : 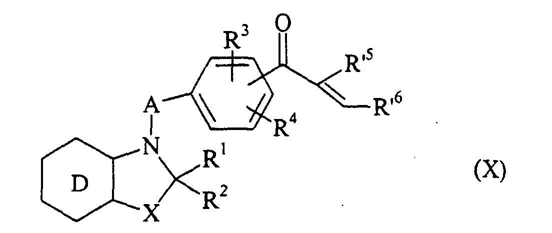 dans laquelle D, X, A, R¹, R², R³, R⁴, R'⁵ et R'⁶ sont définis comme précédemment,
   que l'on soumet à l'action d'un agent réducteur comme Et₃SiH par exemple, pour conduire au composé de formule (I/e), cas particulier des composés de formule (I): 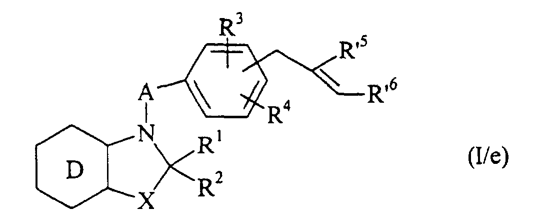 dans laquelle D, X, A, R¹, R², R³, R⁴, R'⁵ et R'⁶ sont définis comme précédemment,
   les composés de formule (I/a), (I/b), (I/c), (I/d) ou (I/e) pour lesquels R'⁵ et R'⁶ représentent des groupements esters pouvant être saponifiés totalement ou partiellement pour conduire aux composés gem-dicarboxylés ou hémicarboxylés correspondants de formule (I/f), cas particulier des composés de formule (I): 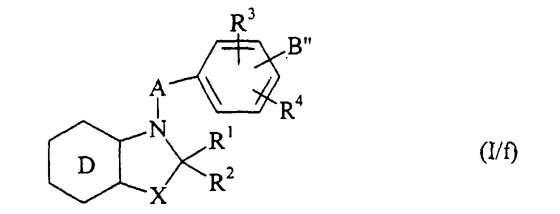 dans laquelle D, X, A, R¹, R², R³ et R⁴ sont tels que définis précédemment et B" représente un groupement tel que défini précédemment pour B dans lequel R⁵ et/ou R⁶ représentent un groupement COOH,
   (les composés de formule (I/f) pour lesquels B" représente un groupement -CH=CH-COOH pouvant être obtenus directement à partir du composé de formule (VI) par condensation de l'acide malonique dans des conditions décarboxylantes et peuvent être réduits pour conduire aux composés de formule (I/f) pour lesquels B" représente un groupement -CH₂-CH₂-COOH),
   les composés de formule (I/a) à (I/f) formant l'ensemble des composés de l'invention, et pouvant être purifiés selon une technique classique de séparation, que l'on transforme, si on le souhaite en leurs sels d'addition à un acide ou à une base pharmaceutiquement acceptable et dont on sépare éventuellement les isomères selon une technique classique de séparation.

Les composés de formule (IV) et (V) sont commerciaux ou aisément accessibles à l'homme du métier par des réactions de chimie classiques ou décrites dans la littérature.

Les composés de la présente invention possèdent des propriétés pharmacologiques très intéressantes.

Ces composés montrent notamment une excellente activité dans la réduction des taux de glucose sanguin. Ces propriétés justifient leur application en thérapeutique dans le traitement et/ou la prophylaxie des hyperglycémies, des dyslipidémies et plus particulièrement dans le traitement des diabètes non insulino-dépendants de type Il, l'intolérance au glucose, les désordres reliés au syndrome X (incluant l'hypertension, l'obésité, la résistance à l'insuline, l'athérosclérose, l'hyperlipidémie), les maladies artérielles coronaires et d'autres maladies cardiovasculaires (incluant l'hypertension artérielle, l'insuffisance cardiaque, l'insuffisance veineuse), les maladies rénales (incluant les glomérulonéphrites, les gloméruloscléroses, le syndrôme néphrotique, la néphrosclérose hypertensive), les rétinopathies, les désordres reliés à l'activation des cellules endothéliales, le psoriasis, le syndrôme polycystique ovarien, la démence, les complications diabétiques et l'ostéoporose.

Ils peuvent être utilisés comme inhibiteurs de l'aldose réductase, pour améliorer les fonctions cognitives dans la démence et les complications du diabète, les maladies inflammatoires intestinales, les dystrophies myotoniques, les pancréatites, l'artériosclérose, le xanthome.

L'activité de ces composés est également recommandée pour le traitement et/ou la prophylaxie d'autres maladies incluant le diabète de type I, les hypertriglycéridémies, le syndrome X, la résistance à l'insuline, les dyslipidémies chez le diabétique, les hyperlipidémies, l'hypercholestérolémie, l'hypertension artérielle, l'insuffisance cardiaque, les maladies cardiovasculaires notamment l'athérosclérose.

De plus, ces composés sont indiqués pour être utilisés dans la régulation de l'appétit, notamment dans la régulation de la prise de nourriture chez des sujets souffrant de désordres tels que l'obésité, l'anorexie, la boulimie et l'anorexie nerveuse.

Ainsi, ces composés peuvent être utilisés en prévention ou pour le traitement de l'hypercholestérolémie, de l'obésité avec des effets avantageux sur l'hyperlipidémie, l'hyperglycémie, l'ostéoporose, l'intolérance au glucose, la résistance à l'insuline ou les maladies dans lesquelles l'insulino-résistance est un mécanisme physiopathologique secondaire.

L'utilisation de ces composés permet de réduire le cholestérol total, le poids corporel, la résistance à la leptine, le glucose plasmatique, les triglycérides, les LDL, les VLDL ainsi que les acides gras libres plasmatiques. Ils peuvent être utilisés en association avec des inhibiteurs de la HMG CoA réductase, les fibrates, l'acide nicotinique, la cholestyramine, le colestipol ou le probucol, et peuvent être administrés ensemble ou à des périodes différentes pour agir en synergie chez le patient traité.

Ils présentent par ailleurs une activité dans les pathologies cancéreuses et notamment les cancers hormone-dépendants tels le cancer du sein et le cancer du colon, ainsi qu'un effet inhibiteur des processus d'angiogénèse impliqués dans ces pathologies.

Parmi les compositions pharmaceutiques selon l'invention, on pourra citer plus particulièrement celles qui conviennent pour l'administration orale, parentérale, nasale, per ou transcutanée, rectale, perlinguale, oculaire ou respiratoire et notamment les comprimés simples ou dragéifiés, les comprimés sublinguaux, les sachets, les paquets, les gélules, les glossettes, les tablettes, les suppositoires, les crèmes, les pommades, les gels dermiques et les ampoules buvables ou injectables.

La posologie varie selon le sexe, l'âge et le poids du patient, la voie d'administration, la nature de l'indication thérapeutique, ou des traitements éventuellement associés et s'échelonne entre 0,1 mg et 1 g par 24 heures en 1 ou plusieurs prises.

Les exemples suivants illustrent l'invention et ne la limitent en aucune façon. Les préparations suivantes conduisent à des intermédiaires de synthèse utiles dans la préparation de l'invention.

### Préparation 1: 4-[2-(2-Oxo-1,3-benzoxazol-3(2H)-yl)éthoxy]benzaldéhyde

### Stade A : 4-(2-Chloroéthoxy)benzaldéhyde

Solubiliser le 4-hydroxybenzaldéhyde (0,082 mole) dans le diméthylformamide (100 ml) et ajouter le carbonate de potassium (0,164 mole) et le 1-bromo-2-chloroéthane (0,246 mole). Laisser sous agitation magnétique pendant cinq jours à température ambiante. Hydrolyser ensuite le milieu réactionnel dans 500 ml d'eau et alcaliniser avec 3 g de soude en pastilles. Extraire par 2 fois 50 ml d'éther. Sécher la phase organique sur du sulfate de magnésium et évaporer l'éther sous pression réduite. Purifier l'huile obtenue par flash chromatographie avec comme éluant le mélange cyclohexane/éther (9/1). Evaporer ensuite le mélange de solvants sous pression réduite et laisser cristalliser l'huile jaune obtenue et la conserver au dessicateur.
Point de fusion : 38-40°C

### Stade B : 4-[2-(2-Oxo-1,3-benzoxazol-3(2H)-yl)éthoxy]benzaldéhyde

Solubiliser la benzoxazolinone (0,024 mole) dans le diméthylformamide et ajouter le carbonate de potassium (0,047 mole). Chauffer à reflux pendant 20 minutes puis ajouter le composé obtenu au stade A (0,021 mole). Laisser sous agitation magnétique et à reflux pendant 80 minutes. Hydrolyser le milieu réactionnel dans 150 ml d'eau, essorer le précipité obtenu et le recristalliser dans un mélange cyclohexane/toluène (5/5).
Point de fusion : 112-113°C

### Préparation 2 :4-[2-(2-Oxo-1,3-benzothiazol-3(2H)-yl)éthoxy]benzaldéhyde

On procède comme dans la Préparation 1 en remplaçant la benzoxazolinone par la benzothiazolinone.
Point de fusion : 118-120°C

### Préparation 3 :4-[3-(2-Oxo-1,3-benzothiazol-3(2H)-yl)propoxy]benzaldéhyde

On procède comme dans la Préparation 1 en remplaçant au stade A le 1-bromo-2-chloroéthane par le 1-bromo-3-chloropropane et au stade B la benzoxazolinone par la benzothiazolinone.

### Préparation 4: 4-[2-(6-Benzoyl-2-oxo-1,3-benzothiazol-3(2H)-yl)éthoxy]benzaldéhyde

### Stade A : 6-Benzoyl-benzothiazolinone

Broyer la benzothiazolinone (10 g) et l'acide benzoïque (9,7 g) dans un mortier. Dans un ballon, y mettre la préparation et ajouter l'acide polyphosphorique (100 g). Chauffer à 140°C pendant 4 heures sous agitation mécanique. Hydrolyser le milieu réactionnel dans de la glace. Filtrer le précipité obtenu, le laver à l'éther et le recristalliser dans un mélange cyclohexane/toluène (4/6).
Point de fusion : 190°C

### Stade B : 4-[2-(6-Benzoyl-2-oxo-1,3-benzothiazol-3(2H)-yl)éthoxy]benzaldéhyde

On procède comme dans le stade B de la Préparation 1 en remplaçant la benzoxazolinone par le composé obtenu au stade A.
Point de fusion : 135-139°C

### Préparation 5 : 4-[2-(6-Benzoyl-2-oxo-1,3-benzoxazol-3(2H)-yl)éthoxy]benzaldéhyde

On procède comme dans la Préparation 4 à partir de la benzoxazolinone.
Point de fusion : 105-108°C

### Préparation 6: 4-[2-(2-Oxo[1,3]oxazolo[4,5-b]pyridin-3(2H)-yl)éthoxy]benzaldéhyde

On procède comme dans la Préparation 1 en remplaçant la benzoxazolinone par la [ 1,3]oxazolo[4,5-*b*]pyridin-2(3*H*)-one.

### Préparation 7: 4-[2-(1H-Pyrrolo[2,3-b]pyridin-1-yl)éthoxy]benzaldéhyde

On procède comme dans la Préparation 1 en remplaçant la benzoxazolinone par la 1*H*-pyrrolo[2,3-*b*]pyridine.

### Préparation 8 : 4-[2-(1H-Pyrrolo[3,2-c]pyridin-1-yl)éthoxy]benzaldéhyde

On procède comme dans la Préparation 1 en remplaçant la benzoxazolinone par la 1*H*-pyrrolo[3,2-*c*]pyridine.

### EXEMPLE 1 : 2-{4-[2-(2-Oxo-1,3-benzoxazol-3(2H)-yl)éthoxy]benzylidène} malonate de diéthyle

Solubiliser le composé obtenu dans la Préparation 1 (1,20 g) dans le toluène (80 ml) puis ajouter la pipéridine (0,02 ml), l'acide acétique glacial (0,05 ml) et le malonate de diéthyle (0,70 ml). Chauffer à ébullition avec distillation azéotropique de l'eau au moyen d'un appareil Dean-Stark pendant 5 jours. Laisser refroidir puis évaporer le toluène sous pression réduite. Réaliser une flash chromatographie avec comme éluant le dichlorométhane afin de purifier l'huile obtenue. Évaporer le dichlorométhane sous pression réduite. Laisser cristalliser l'huile dans de l'éther, filtrer le solide obtenu et le recristalliser dans un mélange cyclohexane/toluène (5/5).
Point de fusion : 98-100°C

### EXEMPLE 2 : 2-{4-[2-(2-Oxo-1,3-benzoxazol-3(2H)-yl)éthoxy]benzyl}malonate de diéthyle

Solubiliser le composé obtenu dans l'Exemple 1 (6,7 g) dans un mélange dioxane/éthanol absolu (100 ml/20 ml) et ajouter le charbon palladié (0,5 g). Laisser sous agitation magnétique à température ambiante et sous atmosphère d'hydrogène pendant 76 heures. Filtrer le charbon palladié et évaporer le mélange de solvants sous pression réduite. Faire cristalliser l'huile obtenue par trituration dans de l'éther isopropylique. Recristalliser le précipité obtenu dans un mélange cyclohexane/toluène (9/1).
Point de fusion : 68-70°C

### EXEMPLE 3 : Acide 3-éthoxy-3-oxo-2-{4-[2-(2-oxo-1,3-benzoxazol-3(2H)-yl) éthoxy]benzyl}propanoïque

Solubiliser le composé obtenu dans l'Exemple 2 (0,5 g) dans l'éthanol absolu (6,5 ml) et ajouter le tétrahydrofurane (2 ml). Ensuite, refroidir le milieu réactionnel à l'aide d'un bain de glace et ajouter la soude 2N (0,7 ml). Laisser sous agitation magnétique pendant 18 heures à température ambiante. Evaporer le mélange éthanol absolu/tétrahydrofurane sous pression réduite. Reprendre le résidu obtenu par de l'eau et extraire par 2 fois 50 ml d'éther. Acidifier la phase aqueuse avec une solution d'acide chlorhydrique 6N jusqu'à pH = 1 puis extraire par 2 fois 50 ml d'éther. Sécher la phase organique sur du sulfate de magnésium et évaporer l'éther sous pression réduite.
Huile.

### EXEMPLE 4 : Acide 2-{4-[2-(2-oxo-1,3-benzoxazol-3(2H)-yl)éthoxy]benzyl} malonique

Solubiliser le composé obtenu dans l'Exemple 2 (1,5 g) dans le tétrahydrofurane (20 ml). Ajouter la soude 2N (8 ml) au milieu réactionnel refroidi à 0°C par un bain de glace. Laisser sous agitation magnétique et à température ambiante pendant 72 heures. Évaporer le tétrahydrofurane sous pression réduite. Reprendre le résidu obtenu par une solution d'acide chlorhydrique 6N jusqu'à pH = 1 et extraire avec 2 fois 50 ml d'éther. Sécher la phase organique sur du sulfate de magnésium et évaporer l'éther sous pression réduite. Faire cristalliser l'huile obtenue dans l'éther isopropylique et recristalliser le précipité obtenu dans le cyclohexane.
Point de fusion : 118-120°C

### EXEMPLE 5 : 2-{4-[2-(2-Oxo-1,3-benzothiazol-3(2H)-yl)éthoxy]benzylidène} malonate de diéthyle

On procède comme dans l'Exemple 1 à partir du composé obtenu dans la Préparation 2.
Point de fusion : 113-114°C

### EXEMPLE 6 : 2-{4-[2-(2-Oxo-1,3-benzothiazol-3(2H)-yl)éthoxy]benzylidène} malonate de diméthyle

On procède comme dans l'Exemple 1 à partir du composé obtenu dans la Préparation 2 et en remplaçant le malonate de diéthyle par le malonate de diméthyle.
Point de fusion : 144-146°C

### EXEMPLE 7 : 2-{4-[2-(2-Oxo-1,3-benzothiazol-3(2H)-yl)éthoxy]benzyl} malonate de diéthyle

On procède comme dans l'Exemple 2 à partir du composé obtenu dans l'Exemple 5.
Huile.

### EXEMPLE 8 : 2-{4-[2-(2-Oxo-1,3-benzothiazol-3(2H)-yl)éthoxy]benzyl} malonate de diméthyle

Dissoudre le composé obtenu dans l'Exemple 6 (3 g) dans le mélange méthanol/dioxane (50 ml/50 ml) et ajouter le charbon palladié (0,75 g). Laisser à température ambiante sous atmosphère d'hydrogène et sous agitation magnétique pendant 5 heures. Filtrer le charbon palladié et évaporer le mélange méthanol/dioxane sous pression réduite. Cristalliser l'huile obtenue dans l'éther isopropylique.
Point de fusion : 67-70°C

### EXEMPLE 9 : Acide 3-méthoxy-3-oxo-2-{4-[2-(2-oxo-1,3-benzothiazol-3(2H)-yl) éthoxy]benzyl}propanoïque

Dissoudre le composé obtenu dans l'Exemple 8 (0,5 g) dans le méthanol (30 ml) et ajouter la potasse (0,07 g) préalablement dissoute dans le méthanol. Laisser à température ambiante pendant une semaine sous agitation magnétique. Evaporer le méthanol sous pression réduite et acidifier le résidu obtenu par une solution d'acide chlorhydrique 6N jusqu'à pH = 1. Extraire par 2 fois 30 ml d'éther puis évaporer la phase organique sous pression réduite après l'avoir séchée sur du sulfate de magnésium. Faire précipiter le résidu obtenu dans l'éther et filtrer le précipité qui se forme.
Point de fusion : 30-35°C

### EXEMPLE 10 : Acide 3-éthoxy-3-oxo-2-{4-[2-(2-oxo-1,3-benzothiazol-3(2H-yl) éthoxy]benzyl}propanoïque

Solubiliser le composé obtenu dans l'Exemple 7 (0,85 g) dans l'éthanol absolu (20 ml) et refroidir le milieu réactionnel par un bain de glace. Ajouter la potasse (0,10 g) et laisser sous agitation magnétique pendant 90 heures à température ambiante. Evaporer l'éthanol absolu sous pression réduite. Reprendre le résidu par 20 ml d'eau et extraire avec 2 fois 30 ml d'éther. Acidifier la phase aqueuse avec une solution d'acide chlorhydrique jusqu'à pH = 1. Extraire par 2 fois 30 ml d'éther. Sécher la phase organique sur du sulfate de magnésium et évaporer l'éther sous pression réduite.
Huile.

### EXEMPLE 11 : Acide 2-{4-[2-(2-oxo-1,3-benzothiazol-3(2H)-yl)éthoxy]benzyl} malonique

Solubiliser le composé obtenu dans l'Exemple 7 (1 g) dans le mélange éthanol absolu/tétrahydrofurane (13 ml/2 ml). Ajouter la soude 2N (2,5 ml) au milieu réactionnel refroidi à 0°C par un bain de glace. Laisser sous agitation magnétique et à température ambiante pendant 48 heures. Evaporer le mélange éthanol absolu/tétrahydrofurane sous pression réduite. Reprendre le résidu obtenu par une solution d'acide chlorhydrique jusqu'à pH = 1 et extraire avec 2 fois 50 ml d'acétate d'éthyle. Sécher la phase organique sur du sulfate de magnésium et évaporer l'acétate d'éthyle sous pression réduite. Essorer et laver le précipité obtenu avec de l'éther puis le recristalliser dans l'acétonitrile.
Point de fusion : 179-181°C

### EXEMPLE 12 : 2-{4-[3-(2-Oxo-1,3-benzothiazol-3(2H)-yl)propoxy]benzylidène} malonate de diéthyle

On procède comme dans l'Exemple 1 à partir du composé obtenu dans la Préparation 3.

### EXEMPLE 13 : 2-{4-[3-(2-Oxo-1,3-benzothiazol-3(2H)-yl)propoxy]benzyl} malonate de diéthyle

On procède comme dans l'Exemple 2 à partir du composé obtenu dans l'Exemple 12.

### EXEMPLE 14 : Acide 2-{4-[3-(2-oxo-1,3-benzothiazol-3(2H)-yl)propoxy]benzyl} malonique

On procède comme dans l'Exemple 4 à partir du composé obtenu dans l'Exemple 13.

### EXEMPLE 15 : 2-{4-[2-(2-Oxo-1,3-benzothiazol-3(2H)-yl)éthoxy]benzylidène} propanedioate de tert-butyle et de méthyle

On procède comme dans l'Exemple 1 à partir du composé obtenu dans la Préparation 2 et en remplaçant le malonate de diéthyle par le malonate de *tert*-butyle et de méthyle.

### EXEMPLE 16 : 2-(4-[2-(2-Oxo-1,3-benzothiazol-3(2H)-yl)éthoxy]benzyl} malonate de tert-butyle et de méthyle

On procède comme dans l'Exemple 2 à partir du composé obtenu dans l'Exemple 15.
Huile.

### EXEMPLE 17 : 2-{4-[2-(6-Benzoyl-2-oxo-1,3-benzothiazol-3(2H)-yl)éthoxy] benzylidène}malonate de diméthyle

On procède comme dans l'Exemple 1 à partir du composé obtenu dans la Préparation 4 et en remplaçant le malonate de diéthyle par le malonate de diméthyle.
Point de fusion : 167-170°C

### EXEMPLE 18: 2-{4-[2-(6-Benzoyl-2-oxo-1,3-benzothiazol-3(2H)-yl)éthoxy]benzyl} malonate de diméthyle

Solubiliser le composé obtenu dans l'Exemple 17 (3,7 g) dans le mélange méthanol/tétrahydrofurane/dioxane (10 ml/30 ml/30 ml) et ajouter le charbon palladié (0,9 g). Laisser sous agitation magnétique et sous atmosphère d'hydrogène à température ambiante pendant 24 heures. Filtrer le charbon palladié et évaporer le mélange de solvants sous pression réduite. Le produit obtenu est recristallisé dans l'éther isopropylique.
Point de fusion : 65-67°C

### EXEMPLE 19: 2-{4-[2-(6-Benzyl-2-oxo-1,3-benzothiazol-3(2H)-yl)éthoxy]benzyl} malonate de diméthyle

Dissoudre le composé obtenu dans l'Exemple 18 (0,7 g) dans l'acide trifluoroacétique (1,6 ml) puis ajouter le triéthylsilane (0,5 ml). Laisser à température ambiante sous agitation magnétique pendant 2 jours. Hydrolyser le milieu réactionnel puis extraire avec 2 fois 30 ml d'éther. Sécher la phase organique sur du sulfate de magnésium et l'évaporer sous pression réduite. Réaliser une flash chromatographie sur le résidu obtenu avec comme éluant le mélange cyclohexane/acétate d'éthyle (8/2).
Point de fusion : 78-81°C

### EXEMPLE 20: 2-{4-[2-(6-Benzoyl-2-oxo-1,3-benzoxazol-3(2H)-yl)éthoxy] benzylidène}malonate de diméthyle

On procède comme dans l'Exemple 1 à partir du composé obtenu dans la Préparation 5 et en remplaçant le malonate de diéthyle par le malonate de diméthyle.
Point de fusion : 173-176°C

### EXEMPLE 21 : 2-{4-[2-(6-Benzoyl-2-oxo-1,3-benzoxazol-3(2H)-yl)éthoxy]benzyl} malonate de diméthyle

On procède comme dans l'Exemple 18 à partir du composé obtenu dans l'Exemple 20.

### EXEMPLE 22 : 2-{4-[2-(6-Benzyl-2-oxo-1,3-benzoxazol-3(2H}-yl)éthoxy]benzyl} malonate de diméthyle

On procède comme dans l'Exemple 19 à partir du composé obtenu dans l'Exemple 21.
Point de fusion : 73-75°C

### EXEMPLE 23 : Acide 3-{4-[2-(2-oxo-1,3-benzoxazol-3(2H)-yl)éthoxy]phényl}-2-propénoïque

Solubiliser le composé obtenu dans la Préparation 1 (1 g) dans un mélange toluène-dioxane (10 ml/20 ml). Ajouter l'acide malonique (0,92 g) et l'α-picoline (0,87 ml). Laisser le mélange sous agitation magnétique dans un bain d'huile à 70-80°C pendant 72 heures. Évaporer sous pression réduite le mélange toluène-dioxane. Reprendre l'huile obtenue par une solution aqueuse de carbonate de sodium 0,1N et extraire avec 2 fois 50 ml d'acétate d'éthyle. Acidifier la phase aqueuse avec une solution d'acide chlorhydrique 6N jusqu'à pH = 1 et essorer le précipité formé. Laver ce dernier à l'éther de pétrole et le recristalliser dans un mélange cyclohexane/toluène (4/6).
Point de fusion : 199-200°C

### EXEMPLE 24 : Acide 3-{4-[2-(2-oxo-1,3-benzoxazol-3(2H)-yl}éthoxy]phényl}-2-propanoïque

Solubiliser le composé obtenu dans l'Exemple 23 (0,45 g) dans le mélange méthanol-dioxane (10 ml/20 ml) et ajouter le charbon palladié (0,14 g). Laisser le mélange sous agitation magnétique à température ambiante et sous atmosphère d'hydrogène pendant 16 heures. Filtrer le charbon palladié et évaporer le mélange méthanol-dioxane sous pression réduite. Faire précipiter l'huile résiduelle dans l'éther, filtrer le précipité obtenu et le recristalliser dans un mélange cyclohexane/toluène (5/5).
Point de fusion : 147-148°C

### EXEMPLE 25 : Acide 3-{4-[2-(2-oxo-1,3-benzothiazol-3(2H)-yl)éthoxy]phényl}-2-propénoïque

On procède comme dans l'Exemple 23 à partir du composé obtenu dans la Préparation 2 mais après avoir acidifié la phase aqueuse avec une solution d'acide chlorhydrique 6N jusqu'à pH = 1, extraire avec 2 fois 50 ml d'acétate d'éthyle. Sécher la phase organique sur du sulfate de magnésium et évaporer l'acétate d'éthyle sous pression réduite. Essorer le précipité solide obtenu, le laver avec de l'éther et le recristalliser dans un mélange cyclohexane/toluène (8/2).
Point de fusion : 199-201°C

### EXEMPLE 26 : Acide 3-{4-[2-(2-oxo-1,3-benzothiazol-3(2H)-yl)éthoxy]phényl}-2- propanoïque

On procède comme dans l'Exemple 24 à partir du composé obtenu dans l'Exemple 25 mais après évaporation des solvants sous pression réduite, essorer le précipité solide obtenu avec de l'éther et le recristalliser dans un mélange cyclohexane/toluène (7/3).
Point de fusion : 168-170°C

### EXEMPLE 27 : 2-{4-[2-(2-Oxo[1,3]oxazolo]4,5-b]pyridin-3(2H)-yl)éthoxyl] benzylidène}malonate de diméthyle

On procède comme dans l'Exemple 1 à partir du composé obtenu dans la Préparation 6 et en remplaçant le malonate de diéthyle par le malonate de diméthyle.

### EXEMPLE 28 : 2-{4-[2-(2-Oxo[1,3]oxazolo[4,5-b]pyridin-3(2H)-yl)éthoxy] benzyl}malonate de diméthyle

On procède comme dans l'Exemple 18 à partir du composé obtenu dans l'Exemple 27.
Huile.

### EXEMPLE 29 : 2-{4-[2-(1H-Pyrrolo[2,3-b]pyridin-1-yl)éthoxy]benzylidène}malonate de diméthyle

On procède comme dans l'Exemple 1 à partir du composé obtenu dans la Préparation 7 et en remplaçant le malonate de diéthyle par le malonate de diméthyle.

### EXEMPLE 30 : 2-{4-[2-(1H-Pyrrolo[2,3-b]pyridin-1-yl)éthoxy]benzyl}malonate de diméthyle

On procède comme dans l'Exemple 18 à partir du composé obtenu dans l'Exemple 29.
Huile.

### EXEMPLE 31 : 3-{4-[2-(6-Benzoyl-2-oxo-1,3-benzothiazol-3(2H)-yl)éthoxy]phényl}-2-propénoate de méthyle

Sous atmosphère d'azote, 1,8 éq d'hydrure de sodium sont dissous dans du THF. Puis 1,4 éq de méthylphosphonoacétate sont ajoutés goutte à goutte à 0°C. Le milieu réactionnel est agité 15 à 20 minutes puis 1 éq du composé obtenu dans la Préparation 4 sont ajoutés. Après 24 heures, le milieu est hydrolysé avec 100 ml d'eau, filtré, et le précipité obtenu est recristallisé dans le méthanol pour conduire au produit du titre.
Point de fusion : 144-146°C

### EXEMPLE 32 : 3-{4-[2-(6-Benzoyl-2-oxo-1,3-benzothiazol-3(2H)-yl)éthoxy]phényl} propanoate de méthyle

Le composé obtenu dans l'Exemple 31 est mis en solution dans un mélange méthanol/dioxane, puis une quantité catalytique de charbon palladié est ajoutée. La réaction est agitée à température ambiante sous atmosphère d'hydrogène 6 à 15 heures. Le charbon est ensuite filtré et les solvants sont évaporés sous vide. Le résidu obtenu est recristallisé dans le méthanol pour conduire au produit du titre.
Point de fusion : 74-77°C

### EXEMPLE 33 : 2-{4-[2-(6-Hydroxy(phényl)méthyl]-2-oxo-1,3-benzoxazol-3(2H)-yl)éthoxy]benzyl}malonate de diméthyle

Le composé obtenu dans l'Exemple 21 est dissous dans le méthanol et 0,25 éq de borohydrure de sodium sont ajoutés. Le milieu réactionnel est agité à température ambiante pendant 24 heures, puis le méthanol est évaporé sous vide et le résidu obtenu est purifié sur chromatographie flash avec un mélange acétate d'éthyle/cyclohexane comme éluant. Le composé du titre ainsi obtenu est recristallisé dans l'éther isopropylique.
Point de fusion : 84-87°C

### EXEMPLE 34 : 3-{4-[2-(6-Benzoyl-2-oxo-1,3-benzoxazol-3(2H)-yl)éthoxy]phényl}-2-propénoate de méthyle

On procède comme dans l'Exemple 31 en remplaçant le composé obtenu dans la Préparation 4 par le composé obtenu dans la Préparation 5.
Point de fusion : 170-173°C

### EXEMPLE 35 : 3-{4-[2-(6-Benzoyl-2-oxo-1,3-benzoxazol-3(2H)-yl)éthoxy]phényl} propanoate de méthyle

On procède comme dans l'exemple 32 à partir du composé obtenu dans l'Exemple 34.
Point de fusion : 100-103°C

### EXEMPLE 36 : 2-{4-[2-(6-[Hydroxy(phényl)méthyl]-2-oxo-1,3-benzothiazol-3(2H)-yl) éthoxy]benzyl}malonate de diméthyle

On procède comme dans l'Exemple 33 à partir du composé obtenu dans l'Exemple 18.
Point de fusion : 153-156°C

### EXEMPLE 37 : 2-{4-[2-(6-(2-Chlorobenzoyl)-2-oxo-1,3-benzothiazol-3(2H)-yl) éthoxy]benzylidène}malonate de diméthyle

### Stade A : 6-(2-Chlorobenzoyl)-1,3-benzothiazol-2(3H)-one

On procède comme dans le stade A de la Préparation 4 en remplaçant l'acide benzoïque par l'acide 2-chlorobenzoïque.

### Stade B : 2-{4-[2-(6-(2-Chlorobenzoyl)-2-oxo-1,3-benzothiazol-3(2H) yl)éthoxy] benzylidène}malonate de diméthyle

Le composé obtenu au stade A est dissous dans du DMF et 2 éq de carbonate de potassium sont ajoutés. Après 20 minutes d'agitation à 80°C, 1,2 éq de 2-[4-(2-chloroéthoxy) benzylidène]malonate de diméthyle sont ajoutés et la réaction est chauffée pendant 12 heures. Le milieu réactionnel est ensuite hydrolysé dans l'eau, filtré et le précipité obtenu est recristallisé dans du méthanol pour conduire au produit du titre.
Point de fusion : 145-149°C

### EXEMPLE 38 : 2-(4-[2-(6-[(Méthoxyimino)(phényl)méthyl]-2-oxo-1,3-benzothiazol-3(2H)-yl)éthoxy]benzyl}malonate de diméthyle

Le composé obtenu dans l'Exemple 18 est dissous dans un minimum de méthanol puis sont successivement ajoutés 4 éq d'O-méthylhydroxylamine préalablement dissous dans quelques millilitres d'eau et 4,5 éq de pyridine. Le milieu réactionnel est chauffé à reflux pendant 24 heures, puis hydrolysé dans l'eau, filtré, et le précipité obtenu est recristallisé dans le méthanol.
Point de fusion : 68-72°C

### EXEMPLE 39 : 2-{4-[2-(2-Oxo-6-(3-pyridinylcarbonyl)-1,3-benzothiazol-3(2H)-yl) éthoxy]benzylidène}malonate de diméthyle

On procède comme dans l'Exemple 37 en remplaçant au stade A l'acide 2-chlorobenzoïque par l'acide nicotinique.
Le solvant de recristallisation est l'éthanol absolu.
Point de fusion : 130-134°C

### EXEMPLE 40 : 2-{4-[2-(6-(2-Chlorobenzoyl)-2-oxo-1,3-benzothiazol-3(2H)-yl)éthoxy] benzyl}malonate de diméthyle

Le composé obtenu dans l'Exemple 37 est dissous dans un mélange de solvants méthanol/THF/dioxane puis une quantité catalytique de charbon palladié est ajoutée et la réaction est agitée à température ambiante sous atmosphère d'hydrogène. Le charbon palladié est ensuite filtré et les solvants sont évaporés sous pression réduite. Le résidu obtenu est repris par l'éther isopropylique et laissé sous agitation magnétique. Le précipité obtenu est filtré et recristallisé dans du méthanol.
Point de fusion : 104-108°C

### EXEMPLE 41 : 2-{4-[2-(6-(3-Chlorobenzoyl)-2-oxo-1,3-benzothiazol-3(2H)-yl)éthoxy] benzylidène}malonate de diméthyle

On procède comme dans l'Exemple 37 en remplaçant au stade A l'acide 2-chlorobenzoïque par l'acide 3-chlorobenzoïque.
Point de fusion : 234-238°C

### EXEMPLE 42 : 2-{4-[2-(6-([1,1'-Biphényl]-4-ylcarbonyl)-2-oxo-1,3-benzothiazol-3(2H)-yl)éthoxy]benzylidène}malonate de diméthyle

On procède comme dans l'Exemple 37 en remplaçant au stade A l'acide 2-chlorobenzoïque par l'acide [1,1'-biphényl]-4-carboxylique.
Point de fusion : 168°C

### EXEMPLE 43 : 2-{4-[2-(6-(4-Chlorobenzoyl)-2-oxo-1,3-benzothiazol-3(2H)-yl)éthoxy]benzylidène}malonate de diméthyle

On procède comme dans l'Exemple 37 en remplaçant au stade A l'acide 2-chlorobenzoïque par l'acide 4-chlorobenzoïque.
Point de fusion : 234-238°C

### EXEMPLE 44: 2-{4-[2-(6-[(Hydroxyimino)(phényl)méthyl]-2-oxo-1,3-benzothiazol-3(2H)-yl)éthoxy]benzyl}malonate de diméthyle

On procède comme dans l'Exemple 38 en remplaçant l'O-méthylhydroxylamine par le chlorhydrate de l'hydroxylamine.
Point de fusion : 161-162°C

### EXEMPLE 45 : 2-{4-[2-(6-([1,1'-Biphényl]-4-ylcarbonyl)-2-oxo-1,3-benzothiazol-3(2H)-yl)éthoxy]benzyl}malonate de diméthyle

On procède comme dans l'Exemple 40 à partir du composé obtenu dans l'Exemple 42.
Point de fusion : 95-96°C

### EXEMPLE 46 : 2-{4-[2-(6-Butyryl-2-oxo-1,3-benzothiazol-3(2H)-yl)éthoxy] benzylidène}malonate de diméthyle

On procède comme dans l'Exemple 37 en remplaçant au stade A l'acide 2-chlorobenzoïque par l'acide butyrique.
Le solvant de recristallisation est un mélange acétate d'éthyle/cyclohexane.
Point de fusion : 154-155°C

### EXEMPLE 47 : 2-{4-[2-(6-(1-Naphtoyl)-2-oxo-1,3-benzothiazol-3(2H)-yl)éthoxy]benzylidène}malonate de diméthyle

On procède comme dans l'Exemple 37 en remplaçant au stade A l'acide 2-chlorobenzoïque par l'acide 1-naphtoïque.
Point de fusion : 143-144°C

### EXEMPLE 48 : 2-{4-[2-(6-([1,1'-Biphényl]-4-ylméthyl-2-oxo-1,3-benzothiazol-3(2H)-yl)éthoxy]benzyl}malonate de diméthyle

Le composé obtenu dans l'Exemple 45 est dissous dans 15 à 20 éq d'acide trifluoroacétique et 2,5 éq de triéthylsilane sont ajoutés. Le milieu réactionnel est agité à température ambiante 24 à 72 heures, puis est hydrolysé avec de l'eau et extrait à l'éther. Les phases organiques sont collectées, séchées sur sulfate de magnésium, évaporées sous vide et le résidu obtenu est recristallisé dans de l'acétate d'éthyle pour conduire au produit du titre.
Point de fusion : 122°C

### EXEMPLE 49 : 2-{4-[2-(6-(1-Naphtoyl)-2-oxo-1,3-benzothiazol-3(2H)-yl)éthoxy] benzyl}malonate de diméthyle

On procède comme dans l'Exemple 40 à partir du composé obtenu dans l'Exemple 47.
Le solvant de recristallisation est l'éther isopropylique.
Point de fusion : 62-63°C

### EXEMPLE 50 : 2-{4-[2-(6-(3-Chlorobenzoyl)-2-oxo-1,3-benzothiazol-3(2H)-yl) éthoxy]benzyl}malonate de diméthyle

On procède comme dans l'Exemple 40 à partir du composé obtenu dans l'Exemple 41.
Le solvant de recristallisation est l'éther isopropylique.
Point de fusion : 90-94°C

### EXEMPLE 51 : 2-{4-[2-(6-(2-Chlorobenzyl)-2-oxo-1,3-benzothiazol-3(2H)-yl)étoxy] benzyl}malonate de diméthyle

On procède comme dans l'Exemple 48 à partir du composé obtenu dans l'Exemple 40.
Le solvant de recristallisation est l'éther de pétrole.
Point de fusion : 92-96°C

### EXEMPLE 52 : 2-{4-[2-(6-(3-Chlorobenzyl)-2-oxo-1,3-benzothiazol-3(2H)-yl)ethoxy] benzyl}malonate de diméthyle

On procède comme dans l'Exemple 48 à partir du composé obtenu dans l'Exemple 50.
Le solvant de recristallisation est l'éther de pétrole.
Point de fusion : 82-86°C

### EXEMPLE 53 : 2-{4-[2-(6-Butyryl-2-oxo-1,3-benzothiazol-3(2H)-yl)éthoxy]benzyl} malonate de diméthyle

On procède comme dans l'Exemple 40 à partir du composé obtenu dans l'Exemple 46.
La purification est réalisée sur silice.
Point de fusion : 74-75°C

### EXEMPLE 54 : 2-{4-[2-(6-(1-Naphtylméthyl)-2-oxo-1,3-benzothiazol-3(2H)-yl) éthoxy]benzyl}malonate de diméthyle

On procède comme dans l'Exemple 48 à partir du composé obtenu dans l'Exemple 49.
Le solvant de recristallisation est le toluène.
Point de fusion : 120-122°C

### EXEMPLE 55 :2-{4-[2-(6-Butyl-2-oxo-1,3-benzothiazol-3(2H)-yl)éthoxy)benzyl} malonate de diméthyle

On procède comme dans l'Exemple 48 à partir du composé obtenu dans l'Exemple 53.
Le solvant de recristallisation est l'hexane.
Point de fusion : 73-74°C

### EXEMPLE 56 : 2-{4-[2-(2-Oxo-6-(3-pyridinylcarbonyl)-1,3-benzothiazol-3(2H-yl) éthoxy]benzyl}malonate de diméthyle

On procède comme dans l'Exemple 40 à partir du composé obtenu dans l'Exemple 39.
La purification est réalisée sur silice.
Point de fusion : 129-134°C

### EXEMPLE 57 : 2-{4-[2-(6-(4-Chlorobenzoyl)-2-oxo-1,3-benzothiazol-3(2H)-yl) éthoxy]benzyl}malonate de diméthyle

On procède comme dans l'Exemple 40 à partir du composé obtenu dans l'Exemple 43.
La purification est réalisée sur silice.
Point de fusion : 80-85°C

### EXEMPLE 58 : Acide 2-{4-[2-(6-benzoyl-2-oxo-1,3-benzothiazol-3(2H)-yl)éthoxy] benzyl}malonique

On procède comme dans l'Exemple 11 à partir du composé obtenu dans l'Exemple 18.

### EXEMPLE 59 :2-{4-[2-(6-(4-Méthoxybenzoyl)-2-oxo-1,3-benzothiazol-3(2H)-yl) éthoxy]benzylidène}malonate de diméthyle

On procède comme dans l'Exemple 37 en remplaçant au stade A l'acide 2-chlorobenzoïque par l'acide 4-méthoxybenzoïque.
Le solvant de recristallisation est l'acétate d'éthyle.
Point de fusion : 144-145°C

### EXEMPLE 60 : 2-{4-[2-(6-(2-Naphtoyl)-2-oxo-1,3-benzothiazol-3(2H)-yl)éthoxy] benzyl}malonate de diméthyle

On procède comme dans l'Exemple 40 à partir du composé obtenu dans l'Exemple 70.
Le solvant de recristallisation est l'hexane.
Point de fusion : 87°C

### EXEMPLE 61 : 2-{4-[2-(6-(4-Méthoxybenzoyl)-2-oxo-1,3-benzothiazol-3(2H)-yl) éthoxy]benzyl}malonate de diméthyle

On procède comme dans l'Exemple 40 à partir du composé obtenu dans l'Exemple 59.
Point de fusion : 114-117°C

### EXEMPLE 62 : 2-{4-[2-(6-(4-Chlorobenzyl)-2-oxo-1,3-benzothiazol-3(2H)-yl) éthoxy]benzyl}malonate de diméthyle

On procède comme dans l'Exemple 48 à partir du composé obtenu dans l'Exemple 57.
La purification est réalisée sur silice.
Point de fusion : 40-45°C

### EXEMPLE 63 : Acide 2-{4-[2-(6-benzoyl-2-oxo-1,3-benzothiazol-3(2H)-yl)éthoxy] benzyl}-3-méthoxy-3-oxopropanoique

On procède comme dans l'Exemple 9 à partir du composé obtenu dans l'Exemple 18.

### EXEMPLE 64 : 2-{4-[2-(6-[(2-Chlorophényl)(méthoxyimino)méthyl]-2-oxo-1,3-benzothiazol-3(2H)-yl}éthoxy]benzlidène}malonate de diméthyle

On procède comme dans l'Exemple 68 à partir du composé obtenu dans l'Exemple 37.
Le solvant de recristallisation est le méthanol.
Point de fusion : décomposition à 142-146°C

### EXEMPLE 65 : 2-{4-[2-(6-[(3-Chlorophényl)(méthoxyimino)méthyl]-2-oxo-1,3-benzothiazol-3(2H)-yl)éthoxy]benzyl}malonate de diméthyle

On procède comme dans l'Exemple 38 à partir du composé obtenu dans l'Exemple 50.
Point de fusion : décomposition à 121-125°C

### EXEMPLE 66 : 2-{4-[2-(6-(4-Méthoxyhenzyl)-2-oxo-1,3-benzothiazol-3(2H)-yl)éthoxy]benzyl}malonate de diméthyle

On procède comme dans l'Exemple 48 à partir du composé obtenu dans l'Exemple 61.
Le solvant de recristallisation est le méthanol.
Point de fusion : 112-113°C

### EXEMPLE 67 : 2-{4-[2-(6-(2-Naphtylméthyl)-2-oxo-1,3-benzothiazol-3(2H)-yl) éthoxy]benzyl}malonate de diméthyle

On procède comme dans l'Exemple 48 à partir du composé obtenu dans l'Exemple 60.
Le solvant de recristallisation est le méthanol.
Point de fusion : 104-105°C

### EXEMPLE 68 : 2-{4-[2-(6-[(3-Chlorophényl)(méthoxyimino)méthyl]-2-oxo-1,3-benzothiazol-3(2H)-yl)éthoxy]benzylidène}malonate de diméthyle

Le composé obtenu dans l'Exemple 41 est dissous dans un minimum de méthanol puis sont successivement ajoutés 4 éq d'O-méthylhydroxylamine préalablement dissous dans quelques millilitres d'eau et 4,5 éq de pyridine. Le milieu réactionnel est chauffé à reflux pendant 24 heures, puis hydrolysé dans l'eau, filtré, et le précipité obtenu est recristallisé dans l'acétonitrile.
Point de fusion : décomposition à 78-82°C puis >200°C

### EXEMPLE 69 : 2-{4-[2-(6-[(4-Chlorophényl)(méthoxyimino)méthyl]-2-oxo-1,3-benzothiazol-3(2H)-yl)éthoxy]benzylidène}malonate de diméthyle

On procède comme dans l'Exemple 68 à partir du composé obtenu dans l'Exemple 43.
Point de fusion : décomposition à 118-121°C puis 158-162°C

### EXEMPLE 70 : 2-{4-[2-(6-(2-Naphtoyl)-2-oxo-1,3-benzothiazol-3(2H)-yl)éthoxy] benzylidène}malonate de diméthyle

On procède comme dans l'Exemple 37 en remplaçant au stade A l'acide 2-chlorobenzoïque par l'acide 2-naphtoïque.
Le solvant de recristallisation est l'acétate d'éthyle.
Point de fusion : 164°C

### EXEMPLE 71 : 2-{4-[2-(6-[[1,1'-Biphényl]-4-yl(méthoxyimino)méthyl]-2-oxo-1,3-benzothiazol-3(2H)-yl)éthoxy]benzyl}malonate de diméthyle

On procède comme dans l'Exemple 38 à partir du composé obtenu dans l'Exemple 45.
Point de fusion : 63-64°C

### EXEMPLE 72 : 2-{4-[2-(6[[1,1'-Biphényl]-4-yl(méthoxyimino)méthyl]-2-oxo-1,3-benzothiazol-3(2H)-yl)éthoxy]benzylidène} malonate de diméthyle

On procède comme dans l'Exemple 68 à partir du composé obtenu dans l'Exemple 42.
Le solvant de recristallisation est le méthanol.
Point de fusion : décomposition à 131-132°C

### EXEMPLE 73 : 2-{4-[2-(6-[(2-Chlorophényl)(méthoxyimino)méthyl]-2-oxo-1,3-benzothiazol-3(2H)-yl)éthoxy]benzyl}malonate de diméthyle

On procède comme dans l'Exemple 38 à partir du composé obtenu dans l'Exemple 40.
Point de fusion : décomposition à 92-95°C

### EXEMPLE 74 : Acide 3-méthoxy-3-oxo-2-{4-[2-(1H-pyrrolo[2,3-b]pyridin-1-yl) éthoxy]benzyl}propanoique

Le composé obtenu dans l'Exemple 30 est dissous dans 5 ml de méthanol et 3 ml de THF. Le milieu est refroidi à 0°C et 0,44 ml d'une solution de soude 2M sont ajoutés goutte à goutte. Après 1 heure 30 minutes d'agitation à température ambiante, les solvants sont évaporés sous pression réduite, le résidu obtenu est repris avec de l'eau et extrait à l'acétate d'éthyle. La phase aqueuse est acidifiée avec une solution d'acide chlorhydrique 2M et extraite à l'acétate d'éthyle. La phase organique est séchée sur sulfate de magnésium, filtrée et évaporée sous pression réduite. Le résidu obtenu est repris avec un minimum d'acétate d'éthyle, et précipité avec du pentane, pour conduire, après filtration au produit du titre.
Point de fusion : 120-122°C

### EXEMPLE 75 : 2-{4-[2-(6-(Benzoylamino)-2-oxo-1,3-benzothiazol-3(2H)-yl)éthoxy] benzyl}malonate de diméthyle

On procède comme dans l'Exemple 40 à partir du composé obtenu ans l'Exemple 76.
Point de fusion : 90-94°C

### EXEMPLE 76 : 2-{4-[2-(6-(Benzoylamino)-2-oxo-1,3-benzothiazol-3(2H)-yl)éthoxy] benzylidène}malonate de diméthyle

On procède comme dans l'Exemple 37.
Point de fusion : 186-190°C

### EXEMPLE 77 : Acide 3-{4-[2-(6-Benzyl-2-oxo-1,3-benzothiazol-3(2H)-yl)éthoxy] phényl}propanoique

Le composé obtenu dans l'Exemple 78 est dissous dans un mélange méthanol/THF et 1,2 éq de soude sont ajoutés goutte à goutte. Le milieu réactionnel est agité à température ambiante pendant 24 heures puis est évaporé et le résidu obtenu est repris dans de l'eau, extrait 2 fois à l'éther et la phase aqueuse est acidifiée avec une solution d'acide chlorhydrique 6N. Le précipité obtenu est filtré et recristallisé dans du méthanol pour conduire au produit du titre.
Point de fusion : 158-163°C

### EXEMPLE 78 : 3-{4-[2-(6-Benzyl-2-oxo-1,3-benzothiazol-3(2H)-yl)éthoxy]phényl} propanoate de méthyle

On procède comme dans l'Exemple 48 à partir du composé obtenu dans l'Exemple 32.
Le solvant de recristallisation est le méthanol.
Point de fusion : 71-75°C

### EXEMPLE 79 :3-{4-[2-(6-[(Méthoxyimino)(phényl)méthyl]-2-oxo-1,3-benzothiazol-3(2H)-yl)éthoxy]phényl}propanoate de méthyle

On procède comme dans l'Exemple 38 à partir du composé obtenu dans l'Exemple 32.

### EXEMPLE 80 : Acide 3-{4-[2-(6-benzoyl-2-oxo-1,3-benzothiazol-3(2H)-yl)éthoxy] phényl}propanoique

On procède comme dan l'Exemple 77 à partir du composé obtenu dans l'Exemple 32.
Le solvant de recristallisation est un mélange toluène/cyclohexane.
Point de fusion : 169-173°C

### EXEMPLE 81 : Acide 2-{4-[2-(1H-pyrrolo[2,3-b]pyridin-1-yl)éthoxy]benzyl} malonique

Le composé obtenu dans l'Exemple 30 (274 mg) est dissous dans 5 ml de méthanol, 2,5 ml de THF et 5 ml d'eau. Une solution de soude 2M est ajoutée (1,8 ml) et le milieu réactionnel est agité 4 jours à température ambiante. Les solvants sont ensuite évaporés sous pression réduite, le résidu obtenu est repris avec de l'eau et extrait à l'acétate d'éthyle. La phase aqueuse est refroidie à 0°C, acidifiée avec une solution d'acide chlorhydrique 2M, et le solide obtenu filtré.
Point de fusion : 182-184°C

### EXEMPLE 82 : 2-{4-[2-(6-Benzoyl-2-oxo-1,3-benzothiazol-3(2H)-yl)éthoxy]benzyl}-3-(méthylamino)-3-oxopropanoate de méthyle

Le composé obtenu dans l'Exemple 63 est dissous dans du dichlorométhane anhydre puis 1,5 éq de chlorure de thionyle sont ajoutés et le milieu est porté à reflux pendant 1 heure 30 minutes. Le milieu réactionnel est ensuite refroidi dans un bain de glace et 1,2 éq de méthylamine sont ajoutés par le haut du réfrigérant. Le milieu réactionnel est agité pendant une heure puis est extrait avec du dichlorométhane. Les phases organiques sont lavées avec une solution de carbonate de sodium, à l'eau, puis filtrées, séchées sur sulfate de magnésium et évaporées sous pression réduite. Le résidu obtenu est purifié sur chromatographie flash avec un mélange acétate d'éthyle/cyclohexane comme éluant. Le produit du titre obtenu est recristallisé dans un mélange toluène/cyclohexane.
Point de fusion : 162-164°C

### EXEMPLE 83 : 2-{4-[2-(6-(Anilinocarbonyl)-2-oxo-1,3-benzothiazol-3(2H)-yl)éthoxy] benzyl}malonate de diméthyle

On procède comme dans l'Exemple 40 à partir du composé obtenu dans l'Exemple 84.
Point de fusion : 104-109°C

### EXEMPLE 84 : 2-{4-[2-(6-(Anilinocarbonyl)-2-oxo-1,3-benzothiazol-3(2H)-yl)éthoxy] benzylidène}malonate de diméthyle

On procède comme dans l'Exemple 37.
Point de fusion : 102-105°C

### EXEMPLE 85 : 2-{4-[2-(2-Oxo-6-[2-phényléthényl]-1,3-benzothiazol-3(2H)-yl)éthoxy] benzylidène}malonate de diméthyle

### Stade A : 6-Nitro-1,3-benzothiazol-2(3H)-one

0,1 mole de benzothiazolinone sont dissoutes dans 100 ml d'anhydride acétique et refroidies à -10°C. 10 ml d'acide nitrique sont ensuite ajoutés goutte à goutte et la réaction est agitée pendant 2 heures. Le précipité formé est filtré et lavé à l'eau puis purifié par chromatographie (éluant CH₂Cl₂ puis AcOEt) pour conduire au produit du titre qui est recristallisé dans l'éthanol.
Point de fusion : 248-253°C

### Stade B : 6-Amino-1,3-benzothiazol-2(3H)-one

Le composé obtenu au stade A (0,036 mole) est dissout dans 90 ml de méthanol puis 2,5 g de palladium sur charbon et 18 g de formiate d'ammonium sont successivement ajoutés et le milieu porté à reflux pendant 18 heures. 40 ml de dioxane sont ajoutés et le reflux est maintenu pendant 24 heures. Le palladium sur charbon est ensuite filtré. Le milieu réactionnel est concentré, le précipité obtenu filtré et le filtrat évaporé sous pression réduite. Le résidu obtenu est repris dans une solution d'acide chlorhydrique 1N et extrait avec 2 x 50 ml d'acétate d'éthyle. La phase aqueuse est alcalinisée avec une solution de carbonate de potassium 10 % et extraite avec 2 x 50 ml d'acétate d'éthyle. La phase organique est séchée puis évaporée sous pression réduite et le précipité obtenu est recristallisé dans l'acétonitrile.
Point de fusion : 220-224°C

### Stade C : 6-(2-Phényléthényl)-1,3-benzothiazol-2(3H)-one

Le composé obtenu au stade B (1 g) est dissout dans HBF₄ (10 ml) et le milieu est refroidi à 0°C. 0,44 g de NaNO₂ préalablement dissouts dans l'eau sont ensuite ajoutés et la réaction agitée à 0°C pendant 1 heure. Le précipité obtenu est filtré et lavé à l'éther, broyé avec BF₃ (4,5 mmoles) et de l'acétate de palladium (0,2 mmoles), puis placé sous atmosphère d'azote avant d'ajouter 15,1 ml de dioxane anhydre. Le milieu réactionnel est agité 5 heures à température ambiante puis une hydrolyse est réalisée avec 50 ml d'eau et une extraction réalisée avec 2 x 30 ml d'éther. La phase organique est séchée sur MgSO₄ puis évaporée sous pression réduite. Le précipité obtenu est lavé à l'éther isopropylique et recristallisé dans le méthanol.
Point de fusion : 179°C

### Stade D : 2-{4-[2-(2-Oxo-6-[2-phényléthényl]-1,3-benzothiazol-3(2H)-yl)éthoxy] benzylidène}malonate de diméthyle

On procède comme au stade B de l'Exemple 37 à partir du composé obtenu au stade C.
Point de fusion : 90°C

### EXEMPLE 86 : 2-(4-{[2-(2-Oxo-1,3-benzothiazol-3(2H)-yl}éthyl]amino}benzylidène) malonate de diméthyle

On procède comme au stade B de l'Exemple 37 à partir de la thiazolinone et en remplaçant le 2-{4-[2-chloroéthoxy]benzylidène}malonate de diméthyle par le 2-{4-[(2-chloroéthyl) amino]benzylidène}malonate de diméthyle.

### EXEMPLE 87 : 2-(4-{[2-(6-Benzoyl-2-oxo-1,3-benzothiazol-3(2H)-yl)éthyl]amino} benzylidène)malonate de diméthyle

On procède comme au stade B de l'Exemple 37 à partir du composé obtenu au stade A de la Préparation 4 et en remplaçant le 2-{4-[2-chloroéthoxy]benzylidène} malonate de diméthyle par le 2-{4-[(2-chloroéthyl)amino]benzylidène}malonate de diméthyle.

### EXEMPLE 88 : 2-(4-{[2-(6-Benzoyl-2-oxo-1,3-benzothiazol-3(2H)-yl)éthyl]amino} benzyl)malonate de diméthyle

On procède comme dans l'Exemple 40 à partir du composé obtenu dans l'Exemple 87.

### EXEMPLE 89 : 2-{4-[2-(2-Oxo-6-[2-phényléthényl]-1,3-benzothiazol-3(2H)-yl) éthoxy]benzyl}malonate de diméthyle

On procède comme dans le stade B de l'Exemple 37 à partir du composé obtenu dans l'Exemple 85 en remplaçant le 2-[4-(2-chloroéthoxy)benzylidène]malonate de diméthyle par le 2-[4-(2-chloroéthoxy)benzyl]malonate de diméthyle.
Point de fusion : 103-107°C

### EXEMPLE 90 : 2-{4-[2-(6-(3-Méthylbenzoyl)-2-oxo-1,3-benzothiazol-3(2H)-yl) éthoxy]benzylidène}malonate de diméthyle

On procède comme dans l'Exemple 37 en remplaçant au stade A l'acide 2-chlorobenzoïque par l'acide 3-méthylbenzoïque.
Point de fusion : 153-154°C

### EXEMPLE 91 : 2-Benzoyl-3-{4-[2-(6-benzoyl-2-oxo-1,3-benzothiazol-3(2H)-yl)éthoxy] phényl}-2-propénoate d'éthyle

Un mélange constitué de 5 g du composé obtenu dans la Préparation 4, 2,14 ml de benzoylacétate d'éthyle, 0,05 éq de pyridine, 0,15 ml d'acide acétique glacial dans 200 ml de toluène anhydre est porté à reflux pendant 40 heures, l'eau formée étant éliminée par distillation azéotropique grâce à un Dean-Stark. Le milieu réactionnel est ensuite filtré et le filtrat est évaporé sous pression réduite. Le produit du titre est purifié par chromatographie (éluant AcOEt/cyclohexane: 3/7), repris par de l'éther diisopropylique et recristallisé dans du toluène.
Point de fusion : 189-190°C

### EXEMPLE 92 : 2-{4-[2-(6-Benzoyl-2-oxo-1,3-benzothiazol-3(2H)-yl)éthoxy]benzyl}-3-oxo-3-phénylpropanoate d'éthyle

Le composé obtenu dans l'Exemple 91 est dissout dans 45 ml d'un mélange EtOH95/Dioxane/THF (1/4/4) puis placé sous vide et sous atmosphère d'hydrogène. Le milieu réactionnel est agité pendant 4 heures 30 puis filtré et le filtrat évaporé sous pression réduite. Le produit du titre est purifié par chromatographie (éluant AcOEt/cyclohexane : 3/7), repris par de l'éther diisopropylique et recristallisé dans du toluène/cyclohexane.
Point de fusion : 93-95°C

### EXEMPLE 93 : 2-{4-[2-(1H-Pyrrolo[3,2-c]pyridin-1-yl)éthoxy]benzylidène}malonate de diméthyle

On procède comme dans l'Exemple 1 à partir du composé obtenu dans la Préparation 8 et en remplaçant le malonate de diéthyle par le malonate de diméthyle.

### EXEMPLE 94 : 2-{4-[2-(1H-Pyrrolo[3,2-c]pyridin-1-yl)éthoxy]benzyl}malonate de diméthyle

On procède comme dans l'Exemple 18 à partir du composé obtenu dans l'Exemple 93.

### EXEMPLE 95 : Acide 3-méthoxy-3-oxo-2-{4-(2-(1H-pyrrolo[3,2-c]pyridin-1-yl) éthoxy]benzyl propanoïque

On procède comme dans l'Exemple 74 à partir du composé obtenu dans l'Exemple 94.

### EXEMPLE 96 : Acide 2-{4-[2-(1H pyrrolo[3,2-c]pyridin-1-yl)éthoxy]benzyl} malonique

On procède comme dans l'Exemple 81 à partir du composé obtenu dans l'Exemple 94.

### EXEMPLE 97 : 2-{4-[2-(6-Benzyl-2-oxo-1,3-benzothiazol-3(2H)-yl)éthoxy]benzyl}-3-[2-(méthylamino)phényl]-3-oxopropanoate de méthyle

On procède comme dans l'Exemple 37 en remplaçant au stade A l'acide 2-chlorobenzoïque par l'acide benzoïque et en remplaçant au stade B le 2-[4-(2-chloroéthoxy)benzylidène]malonate de diméthyle par le 2-[4-(2-chloroéthoxy)benzyl]-3-[2-(méthylamino)phényl]-3-oxopropanoate de méthyle.

### EXEMPLE 98 : 3-{4-[2-(1H-Pyrrolo[2,3-b]pyridin-1-yl)éthoxy]phényl}-2-propénoate de méthyle

On procède comme dans l'Exemple 31 en remplaçant le produit obtenu dans la Préparation 4 par le produit obtenu dans la Préparation 7.

### EXEMPLE 99 : 3-{4-[2-(1H-Pyrrolo[2,3-b]pyridin-1-yl)éthoxy]phényl}-propanoate de méthyle

On procède comme dans l'Exemple 32 à partir du composé obtenu dans l'Exemple 98.

### ETUDE PHARMACOLOGIQUE

### Exemple A : Etude de la toxicité aiguë

La toxicité aiguë a été appréciée après administration orale à des lots de 8 souris (26 ± 2 g). Les animaux ont été observés à intervalles réguliers au cours de la première journée et quotidiennement pendant deux semaines suivant le traitement. La DL₅₀, entraînant la mort de 50 % des animaux, a été évaluée et a montré la faible toxicité des composés de l'invention.

### Exemple B : Efficacité dans les modèles génétiques

Des mutations chez des animaux de laboratoire ainsi que des sensibilités différentes à des régimes alimentaires ont permis le développement de modèles animaux présentant des diabètes non insulino-dépendants et des hyperlipidémies associés à l'obésité et à la résistance à l'insuline.
Des modèles génétiques souris (ob/ob) (Diabètes, 1982, 31 (1), 1-6) et rats Zucker (fa/fa) ont été développés par différents laboratoires pour comprendre la physiopathologie de ces maladies et tester l'efficacité de nouveaux composés antidiabétiques (Diabètes, 1983, 32, 830-838).

### Effet antidiabétique et hypolipémiant chez la souris ob/ob

La souris femelle ob/ob (Harlan) âgée de 10 semaines est utilisée pour les tests *in vivo.* Ces animaux sont maintenus sous un cycle lumière-obscurité de 12 heures à 25 °C. Cette souris a une hyperglycémie basale située à 2 g/1. Les animaux sont randomisés par rapport à leur glycémie pour former des groupes de six. Les composés testés par voie intrapéritonéale sont dissous dans un mélange de diméthylsulfoxide (10 %) et de solutol (15 %) pour être administrés à 10 mg/kg sous un volume de 2,5 ml/kg, deux fois par jour pendant quatre jours. Par voie *per os,* les composés sont testés à 30 mg/kg administrés sous un volume de 2,5 ml/kg de HEC 1 %, deux fois par jour pendant quatre jours. Les groupes contrôles reçoivent les solvants dans les mêmes conditions que les groupes traités. L'activité des produits est évaluée par une mesure de la glycémie 24 heures après la dernière administration et par la mesure quotidienne du poids corporel.

Les composés de l'invention montrent une très bonne capacité à réduire la glycémie comparable aux effets obtenus avec la Rosiglitazone, substance de référence, mais avec une variation du poids corporel non significative, alors que dans les mêmes conditions, la Rosiglitazone montre une augmentation de + 4 % significatifs en quatre jours. Par ailleurs, aucun effet secondaire n'a été observé durant les test *in vivo.*

A titre d'Exemple, le composé de l'Exemple 18, administré à 30 mg/kg *per os* réduit significativement la glycémie de 43 %, et de 45 % lorsqu'il est administré *ip* à 10 mg/kg.

### Exemple C : Composition pharmaceutique

| | |
|---|---|
| 1000 comprimés dosés à 5 mg de 2-{4-[2-(6-benzoyl-2-oxo-1,3-benzothiazol-3(2*H*-)yl)éthoxy]benzyl}malonate de diméthyle (Exemple 18) | 5 g |
| Amidon de blé | 20 g |
| Amidon de maïs | 20 g |
| Lactose | 30 g |
| Stéarate de magnésium | 2 g |
| Silice | 1 g |
| Hydroxypropylcellulose | 2 g |

## Revendications

1. Composés de formule (I) : 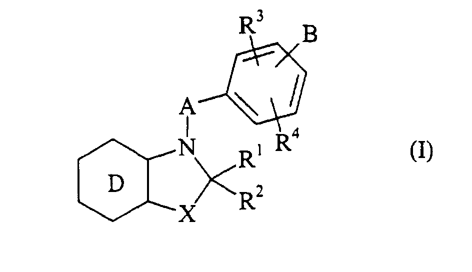 dans laquelle :
• X représente un atome d'oxygène ou de soufre, ou un groupement CH₂ ou 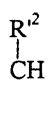 (dans lequel R'² forme avec R² une liaison supplémentaire),
• R¹ et R², identiques ou différents représentent un atome d'hydrogène, un groupement alkyle (C₁-C₆) linéaire ou ramifié, aryle, arylalkyle (C₁-C₆) linéaire ou ramifié, aryloxy, arylalkyloxy (C₁-C₆) linéaire ou ramifié, alkoxy (C₁-C₆) linéaire ou ramifié, hydroxy, amino, alkylamino (C₁-C₆) linéaire ou ramifié ou dialkylamino (C₁-C₆) linéaire ou ramifié,
ou R¹ et R² forment ensemble un groupement oxo, thioxo ou imino,
R² pouvant de plus former avec R'² une liaison supplémentaire,
• A représente une chaîne alkylène (C₁-C₆) dont un groupement CH₂ peut être remplacé par un hétéroatome choisi parmi oxygène ou soufre, ou par un groupement NRₐ (où Rₐ représente un atome d'hydrogène ou un groupement alkyle (C₁-C₆) linéaire ou ramifié), ou par un groupement phénylène ou naphtylène,
• B représente un groupement alkyle (C₁-C₆) linéaire ou ramifié, ou alkényle (C₂-C₆) linéaire ou ramifié, ces groupements étant substitués par un groupement R⁵, par un groupement de formule (II) : 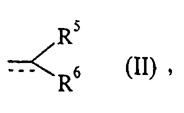 ou par un groupement de formule (III): 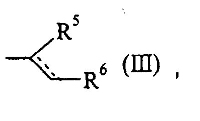 dans lesquels :
- la représentation ---- signifie que la liaison est simple ou double,
- R⁵ représente un groupement 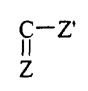
dans lequel Z représente un atome de soufre ou d'oxygène et Z' représente un groupement OR ou NRR',
- et R⁶ représente un groupement 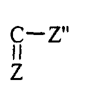
dans lequel Z" représente un groupement Z' ou R,
(où R et R', identiques ou différents, représentent un groupement R" ou - C(Me)₂COOR" où R" représente un atome d'hydrogène ou un groupement alkyle (C₁-C₆) linéaire ou ramifié, alkényle (C₂-C₆) linéaire ou ramifié, alkynyle (C₂-C₆) linéaire ou ramifié, aryle, arylalkyle (C₁-C₆) linéaire ou ramifié, arylalkényle (C₂-C₆) linéaire ou ramifié, arylalkynyle (C₂-C₆) linéaire ou ramifié, hétéroaryle, hétéroarylalkyle (C₁-C₆) linéaire ou ramifié, hétéroarylalkényle (C₂-C₆) linéaire ou ramifié, hétéroarylalkynyle (C₂-C₆) linéaire ou ramifié, cycloalkyle (C₃-C₈), cycloalkyl(C₃-C₈)alkyle(C₁ -C₆) linéaire ou ramifié ou polyhalogénoalkyle (C₁-C₆) linéaire ou ramifié),
• R³ et R⁴, identiques ou différents, représentent un atome d'hydrogène ou d'halogène, ou un groupement R, OR ou NRR' (où R et R' sont tels que définis précédemment), ou R³ et R⁴ forment ensemble avec les atomes de carbone qui les portent, lorsqu'ils sont portés par deux atomes de carbone adjacents, un cycle comportant 5 ou 6 chaînons et pouvant contenir un hétéroatome choisi parmi oxygène, soufre et azote,
• D représente :
un noyau benzénique et dans ce cas X ne peut représenter un groupement 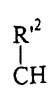 tel que défini précédemment,
ou D représente un noyau pyridinique, pyrazinique, pyrimidinique ou pyridazinique, ces noyaux étant non substitués ou substitués par 1 à 3 groupements, identiques ou différents, choisis parmi R, OR, S(O)ₙR, C(Z)R, 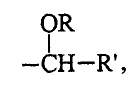 C(Z)OR, NRR', C(Z)NRR', 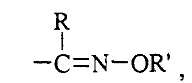 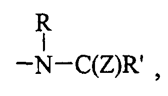 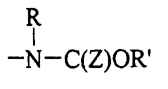 (où R, R' et Z sont tels que définis précédemment et n vaut 0, 1 ou 2), cyano, nitro ou atomes d'halogène,
étant entendu que :
* lorsque A représente un groupement CH₂, B ne peut représenter un groupement alkyle (C₁-C₆) linéaire ou ramifié substitué par un groupement 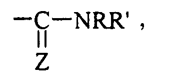
* lorsque les groupements A et B sont en ortho l'un par rapport à l'autre sur le noyau benzénique qui les porte, B ne peut représenter un groupement alkénylène (C₂-C₆) linéaire ou ramifié substitué par un groupement 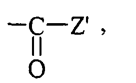
* lorsque A représente un groupement 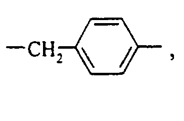 -CH₂-COOH, B ne peut représenter un groupement
* par aryle on entend un groupement phényle, naphtyle ou biphényle, ces groupements pouvant être partiellement hydrogénés,
* par hétéroaryle on entend tout groupement aromatique mono ou bicyclique contenant 5 à 10 chaînons, pouvant être partiellement hydrogéné sur un des cycles dans le cas des hétéroaryles bicycliques, et contenant 1 à 3 hétéroatomes choisis parmi oxygène, azote et soufre,
les groupements aryle et hétéroaryle ainsi définis pouvant être substitués par 1 à 3 groupements choisis parmi alkyle (C₁-C₆) linéaire ou ramifié, alkoxy (C₁-C₆) linéaire ou ramifié, carboxy, formyle, NR_{b}R_{c} (dans lequel R_{b} et R_{c}, identiques ou différents, représentent un atome d'hydrogène, un groupement alkyle (C₁-C₆) linéaire ou ramifié, aryle ou hétéroaryle), ester, amido, nitro, cyano, O-C(Me)₂COOR" (où R" est tel que défini précédemment), ou atomes d'halogène,
leurs énantiomères et diastéréoisomères ainsi que leurs sels d'addition à un acide ou à une base pharmaceutiquement acceptables.

2. Composés de formule (I) selon la revendication 1 pour lesquels R¹ et R² forment un groupement oxo, leurs énantiomères et diastéréoisomères ainsi que leurs sels d'addition à un acide ou à une base pharmaceutiquement acceptables.

3. Composés de formule (I) selon la revendication 1 pour lesquels R³ et R⁴ représentent simultanément un atome d'hydrogène, leurs énantiomères et diastéréoisomères ainsi que leurs sels d'addition à un acide ou à une base pharmaceutiquement acceptables.

4. Composés de formule (I) selon la revendication 1 pour lesquels A représente un groupement alkylène dont un groupement CH₂ peut être remplacé par un hétéroatome, leurs énantiomères et diastéréoisomères ainsi que leurs sels d'addition à un acide ou à une base pharmaceutiquement acceptables.

5. Composés de formule (I) selon la revendication 1 pour lesquels A représente un groupement éthylèneoxy, leurs énantiomères et diastéréoisomères ainsi que leurs sels d'addition à un acide ou à une base pharmaceutiquement acceptables.

6. Composés de formule (I) selon la revendication 1 pour lesquels A représente un groupement alkylène dont un groupement CH₂ peut être remplacé par un groupement phénylène ou naphtylène, leurs énantiomères et diastéréoisomères ainsi que leurs sels d'addition à un acide ou à une base pharmaceutiquement acceptables.

7. Composés de formule (I) selon la revendication 1 pour lesquels X représente un atome d'oxygène ou de soufre, leurs énantiomères et diastéréoisomères ainsi que leurs sels d'addition à un acide ou à une base pharmaceutiquement acceptables.

8. Composés de formule (I) selon la revendication 1 pour lesquels X représente un groupement CHR'² et D représente un noyau pyridinique, leurs énantiomères et diastéréoisomères ainsi que leurs sels d'addition à un acide ou à une base pharmaceutiquement acceptables.

9. Composés de formule (I) selon la revendication 1 pour lesquels B représente un groupement alkyle ou alkényle, ces groupements étant substitués par un groupement de formule (II), leurs énantiomères et diastéréoisomères ainsi que leurs sels d'addition à un acide ou à une base pharmaceutiquement acceptables.

10. Composés de formule (I) selon la revendication 1 pour lesquels B représente un groupement alkyle ou alkényle, ces groupements étant substitués par un groupement R⁵, leurs énantiomères et diastéréoisomères ainsi que leurs sels d'addition à un acide ou à une base pharmaceutiquement acceptables.

11. Composés de formule (I) selon la revendication 1 pour lesquels D représente un noyau benzénique non substitué, leurs énantiomères et diastéréoisomères ainsi que leurs sels d'addition à un acide ou à une base pharmaceutiquement acceptables.

12. Composés de formule (I) selon la revendication 1 pour lesquels D représente un noyau benzénique substitué par un groupement arylcarbonyle, leurs énantiomères et diastéréoisomères ainsi que leurs sels d'addition à un acide ou à une base pharmaceutiquement acceptables.

13. Composés de formule (I) selon la revendication 1 pour lesquels D représente un noyau pyridinique, leurs énantiomères et diastéréoisomères ainsi que leurs sels d'addition à un acide ou à une base pharmaceutiquement acceptables.

14. Composés de formule (I) selon la revendication 1 pour lesquels D représente un noyau pyrazinique, pyrimidinique ou pyridazinique, leurs énantiomères et diastéréoisomères ainsi que leurs sels d'addition à un acide ou à une base pharmaceutiquement acceptables.

15. Composés de formule (I) selon la revendication 1 qui sont le 2-{4-[2-(2-oxo-1,3-benzoxazol-3(2*H*)-yl)éthoxy]benzylidène}malonate de diéthyle, le 2-{4-[2-(2-oxo-1,3-benzothiazol-3(2*H*)-yl)éthoxy]benzylidène}malonate de diéthyle et le 2-{4-[3-(2-oxo-1,3-benzothiazol-3(2*H*)-yl)propoxy]benzylidène}malonate de diéthyle ainsi que leurs sels d'addition à un acide ou à une base pharmaceutiquement acceptables.

16. Composés de formule (I) selon la revendication 1 qui sont le 2-{4-[2-(2-oxo-1,3-benzoxazol-3(2*H*)-yl)éthoxy]benzy}malonate de diéthyle, l'acide 3-éthoxy-3-oxo-2-{4-[2-(2-oxo-1,3-benzoxazol-3(2*H*)-yl)éthoxy]benzyl}propanoïque, l'acide 2-{4-[2-(2-oxo-1,3-benzoxazol-3(2*H*)-yl)éthoxy]benzyl}malonique, le 2-{4-[2-(2-oxo-1,3-benzothiazol-3(2*H*)-yl)éthoxy]benzyl} malonate de diéthyle, le 2-{4-[2-(2-oxo-1,3-benzothiazol-3(2*H*)-yl)éthoxy]benzyl}malonate de diméthyle, l'acide 3-méthoxy-3-oxo-2-{4-[2-(2-oxo-1,3-benzothiazol-3(2*H*)-yl) éthoxy]benzyl}propanoïque, l'acide 3-éthoxy-3-oxo-2-{4-[2-(2-oxo-1,3-benzothiazol-3(2*H*)-yl)éthoxy]benzyl}propanoïque et l'acide 2-{4-[2-(2-oxo-1,3-benzothiazol-3(2*H*)-yl)éthoxy]benzyl}malonique ainsi que leurs sels d'addition à un acide ou à une base pharmaceutiquement acceptables.

17. Composés de formule (I) selon la revendication 1 qui sont le 2-{4-[2-(6-benzoyl-2-oxo-1,3-benzothiazol-3(2*H*)-yl)éthoxy]benzyl}malonate de diméthyle, le 2-{4-[2-(6-benzoyl-2-oxo-1,3-benzoxazol-3(2*H*)-yl)éthoxy]benzyl}malonate de diméthyle, le 2-{4-[2-(6-(2-chlorobenzoyl)-2-oxo-1,3-benzothiazol-3(2*H*)-yl)éthoxy] benzyl}malonate de diméthyle, le 2-{4-[2-(6-([1,1'-biphényl]-4-ylcarbonyl)-2-oxo-1,3-benzothiazol-3(2*H*)-yl)éthoxy]benzyl}malonate de diméthyle, le 2-{4-[2-(6-(1-naphtoyl)-2-oxo-1,3-benzothiazol-3(2*H*)-yl)éthoxy]benzyl}malonate de diméthyle, le 2-{4-[2-(6-(3-chlorobenzoyl)-2-oxo-1,3-benzothiazol-3(2*H*)-yl) éthoxy]benzyl}malonate de diméthyle, le 2-{4-[2-(2-oxo-6-(3-pyridinylcarbonyl)-1,3-benzothiazol-3(2*H*)-yl)éthoxy]benzyl}malonate de diméthyle, le 2-{4-[2-(6-(4-chlorobenzoyl)-2-oxo-1,3-benzothiazol-3(2*H*)-yl)éthoxy]benzyl} malonate de diméthyle, l'acide 2-{4-[2-(6-benzoyl-2-oxo-1,3-benzothiazol-3(2*H*)-yl)éthoxy]benzyl}malonique, le 2-(4-[2-(6-(2-naphtoyl)-2-oxo-1,3-benzothiazol-3(2*H*)-yl)éthoxy]benzyl} malonate de diméthyle, le 2-{4-[2-(6-(4-méthoxybenzoyl)-2-oxo-1,3-benzothiazol-3(2*H*)-yl)éthoxy]benzyl} malonate de diméthyle, l'acide 2-{4-[2-(6-benzoyl-2-oxo-1,3-benzothiazol-3(2*H*)-yl)éthoxy]benzyl}-3-méthoxy-3-oxopropanoique et le 2-{4-[2-(6-benzoyl-2-oxo-1,3-benzothiazol-3{2*H*)-yl)éthoxy] benzyl}-3-(méthylamino)-3-oxopropanoate de méthyle ainsi que leurs sels d'addition à un acide ou à une base pharmaceutiquement acceptables.

18. Composés de formule (I) selon la revendication 1 qui sont le 2-{4-[2-(6-benzyl-2-oxo-1,3-benzothiazol-3(2*H*)-yl)éthoxy]benzyl}malonate de diméthyle, le 2-{4-[2-(6-benzyl-2-oxo-1,3-benzoxazol-3(2*H*)-yl)éthoxy]benzyl}malonate de diméthyle, le 2-{4-[2-(6-(2-chlorobenzyl)-2-oxo-1,3-benzothiazol-3(2*H*)-yl)éthoxy] benzyl}malonate de diméthyle, le 2-{4-[2-(6-(3-chlorobenzyl)-2-oxo-1,3-benzothiazol-3(2*H*)-yl)ethoxy]benzyl}malonate de diméthyle, le 2-{4-[2-(6-(1-naphtylméthyl)-2-oxo-1,3-benzothiazol-3(2*H*)-yl)éthoxy]benzyl}malonate de diméthyle et le 2-{4-[2-(6-(4-chlorobenzyl)-2-oxo-1,3-benzothiazol-3(2*H*)-yl)éthoxy]benzyl}malonate de diméthyle ainsi que leurs sels d'addition à un acide ou à une base pharmaceutiquement acceptables.

19. Composés de formule (I) selon la revendication 1 qui sont le 2-{4-[2-(2-oxo[1,3]oxazolo[4,5-*b*]pyridin-3(2*H*)-yl)éthoxy]benzyl}malonate de diméthyle, le 2-{4-[2-(1*H* pyrrolo[2,3-*b*]pyridin-1-yl)éthoxy]benzyl}malonate de diméthyle et l'acide 2-{4-[2-(1*H*-pyrrolo[2,3-*b*]pyridin-1-yl)éthoxy]benzyl}malonique ainsi que leurs sels d'addition à un acide ou à une base pharmaceutiquement acceptables.

20. Composés de formule (I) selon la revendication 1 qui sont le 2-{4-[2-(6-[hydroxy(phényl)méthyl]-2-oxo-1,3-benzoxazol-3(2*H*)-yl)éthoxy]benzyl}malonate de diméthyle et le 2-{4-[2-(6-[hydroxy(phényl)méthyl]-2-oxo-1,3-benzothiazol-3(2*H*)-yl)éthoxy]benzyl}malonate de diméthyle ainsi que leurs sels d'addition à un acide ou à une base pharmaceutiquement acceptables.

21. Composés de formule (I) selon 1a revendication 1 qui sont le 2-{4-[2-(6-[(méthoxyimino)(phényl)méthyl]-2-oxo-1,3-benzothiazol-3(2*H*)-yl)éthoxy]benzyl} malonate de diméthyle, le 2-{4-[2-(6-[(hydroxyimino)(phényl)méthyl]-2-oxo-1,3-benzothiazol-3(2*H*)-yl)éthoxy]benzyl}malonate de diméthyle, le 2-{4-[2-(6-[(3-chlorophényl)(méthoxyimino)méthyl]-2-oxo-1,3-benzothiazol-3(2*H*)-yl)éthoxy] benzyl}malonate de diméthyle et le 2-{4-[2-(6-[[1,1'-biphényl]-4-yl(méthoxyimino)méthyl]-2-oxo-1,3-benzothiazol-3(2*H*)-yl)éthoxy]benzyl} malonate de diméthyle ainsi que leurs sels d'addition à un acide ou à une base pharmaceutiquement acceptables.

22. Composés de formule (I) selon la revendication 1 qui sont l'acide 3-{4-[3-(2-oxo-1,3-benzoxazol-3(2*H*)-yl)propyl]phényl}-2-propénoïque, l'acide 3-{4-[3-(2-oxo-1,3-benzoxazol-3(2*H*)-yl)propyl]phényl}-2-propanoïque, l'acide 3-{4-[3-(2-oxo-1,3-benzothiazol-3(2*H*)-yl)propyl]phényl}-2-propénoïque, l'acide 3-{4-[3-(2-oxo-1,3-benzothiazol-3(2*H*)-yl)propyl]phényl}-2-propanoïque, le 3-(4-[2-(6-benzoyl-2-oxo-1,3-benzothiazol-3(2*H*)-yl)éthoxy]phényl}propanoate de méthyle, le 3-{4-[2-(6-benzoyl-2-oxo-1,3-benzoxazol-3(2*H*)-yl)éthoxy]phényl}propanoate de méthyle, le 3-{4-[2-(6-benzyl-2-oxo-1,3-benzothiazol-3(2*H*)-yl)éthoxy]phényl}propanoate de méthyle, le 3-{4-[2-(6-[(méthoxyimino)(phényl)méthyl]-2-oxo-1,3-benzothiazol-3(2*H*)-yl)éthoxy]phényl} propanoate de méthyle et l'acide 3-{4-[2-(6-benzoyl-2-oxo-1,3-benzothiazol-3(2*H*)-yl)éthoxy]phényl}propanoique ainsi que leurs sels d'addition à un acide ou à une base pharmaceutiquement acceptables.

23. Procédé de préparation des composés de formule (I) selon la revendication 1 **caractérisé en ce que** l'on utilise comme produit de départ un composé de formule (IV) : 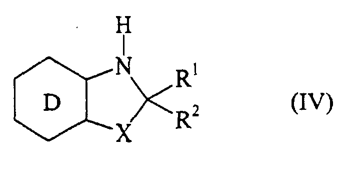 dans laquelle R¹, R², X et D sont tels que définis dans la formule (I),
sur lequel on condense en milieu basique un composé de formule (V) : 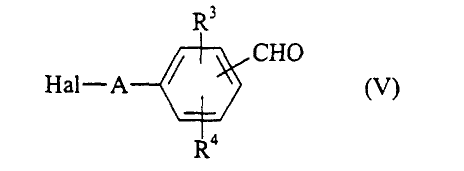 dans laquelle Hal représente un atome d'halogène et A, R³ et R⁴ sont tels que définis dans la formule (I),
pour conduire au composé de formule (VI) : 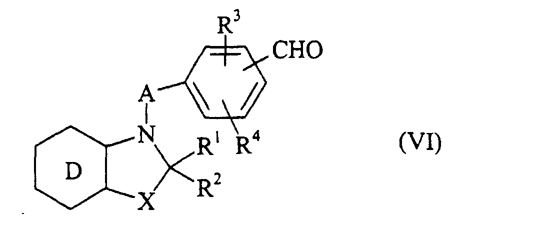 dans laquelle A, D, X, R¹, R², R³ et R⁴ sont définis de la même façon que précédemment,
- sur lequel on condense en milieu basique un composé de formule (VII) : 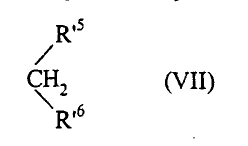
dans laquelle R'⁵ et R'⁶ peuvent prendre toutes les valeurs définies précédemment pour R⁵ et R⁶ à l'exception du groupement COOH,
pour obtenir le composé de formule (I/a), cas particulier des composés de formule (I): 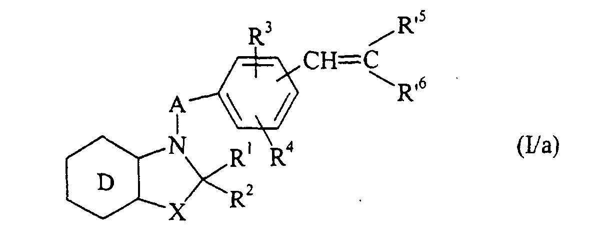 dans laquelle D, A, X, R¹, R², R³, R⁴, R'⁵ et R'⁶ sont définis comme précédemment,
qui est hydrogéné en présence d'un catalyseur comme le palladium sur charbon par exemple pour conduire au composé de formule (I/b), cas particulier des composés de formule (I): 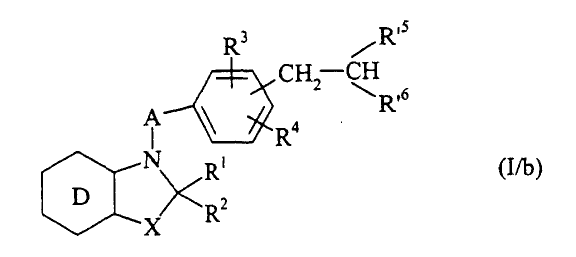 dans laquelle D, A, X, R¹, R², R³, R⁴, R'⁵ et R'⁶ sont tels que définis précédemment,
(les composés de formule (I/a) et (I/b) pouvant être obtenus par condensation directe sur le composé de formule (IV) d'un composé de formule (V') 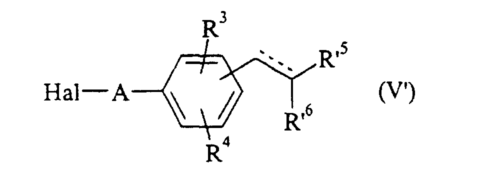 dans laquelle Hal représente un atome d'halogène et A, R³, R⁴, R'⁵ et R'⁶ sont tels que définis précédemment),
- ou composé de formule (VI) sur lequel on condense, dans les conditions d'une réaction de Horner-Errunons le composé phosphonique correspondant d'un dérivé de formule (VIII):
Hal-CH₂-B' (VIII)
dans laquelle Hal représente un atome d'halogène et B' représente un groupement alkyle (C₁-C₅) linéaire ou ramifié ou alkényle (C₂-C₅) linéaire ou ramifié, ces groupements étant substitués par un groupement R'⁵ ou par un groupement de formule (II') 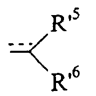 ou par un groupement de formule (III') 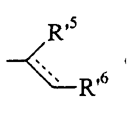 dans lesquels R'⁵ et R'⁶ et la représentation ---- sont tels que définis précédemment,
pour conduire au composé de formule (I/c), cas particulier des composés de formule (I) : 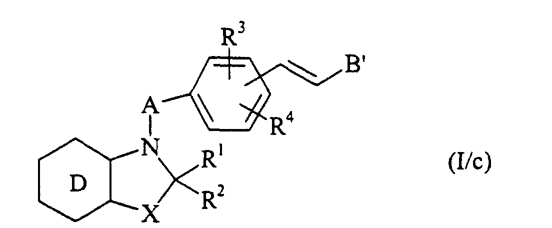 dans laquelle D, X, A, B', R¹, R², R³ et R⁴ sont tels que définis précédemment,
qui peut être soumis à une hydrogénation catalytique, en présence de palladium par exemple pour obtenir le composé de formule (I/d), cas particulier des composés de formule (I): 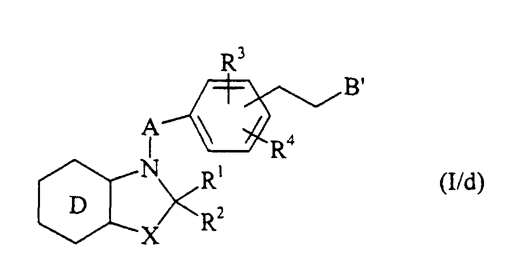 dans laquelle D, A, X, B', R¹, R², R³ et R⁴ sont tels que définis précédemment,
- ou composé de formule (VI) que l'on transforme en chlorure d'acide correspondant sur lequel on condense, en présence d'un dérivé du palladium ou de l'étain par exemple, le composé de formule (IX) : 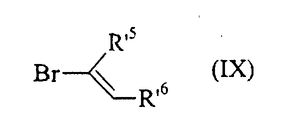 dans laquelle R'⁵ et R'⁶ sont tels que définis précédemment,
pour conduire au composé de formule (X) : 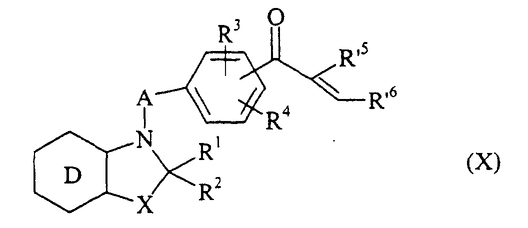 dans laquelle D, X, A, R¹, R², R³, R⁴, R'⁵ et R'⁶ sont définis comme précédemment,
que l'on soumet à l'action d'un agent réducteur comme Et₃SiH par exemple, pour conduire au composé de formule (I/e), cas particulier des composés de formule (I): 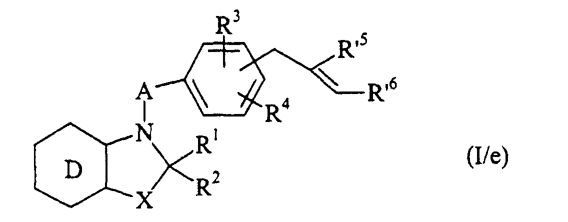 dans laquelle D, X, A, R¹, R², R³, R⁴, R'⁵ et R'⁶ sont définis comme précédemment,
les composés de formule (I/a), (I/b), (I/c), (I/d) ou (I/e) pour lesquels R'⁵ et R'⁶ représentent des groupements esters pouvant être saponifiés totalement ou partiellement pour conduire aux composés gem-dicarboxylés ou hémicarboxylés correspondants de formule (I/f), cas particulier des composés de formule (I): 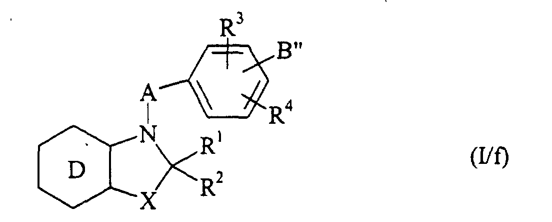 dans laquelle D, X, A, R¹, R², R³ et R⁴ sont tels que définis précédemment et B" représente un groupement tel que défini précédemment pour B dans lequel R⁵ et/ou R⁶ représentent un groupement COOH,
(les composés de formule (I/f) pour lesquels B" représente un groupement -CH=CH-COOH pouvant être obtenus directement à partir du composé de formule (VI) par condensation de l'acide malonique dans des conditions décarboxylantes et peuvent être réduits pour conduire aux composés de formule (I/f) pour lesquels B" représente un groupement -CH₂-CH₂-COOH),
les composés de formule (I/a) à (I/f) formant l'ensemble des composés de l'invention, et pouvant être purifiés selon une technique classique de séparation, que l'on transforme, si on le souhaite en leurs sels d'addition à un acide ou à une base pharmaceutiquement acceptable et dont on sépare éventuellement les isomères selon une technique classique de séparation.

24. Compositions pharmaceutiques contenant comme principe actif au moins un composé de formule (I) selon l'une quelconque des revendications 1 à 22 ou un de ses sels d'addition à un acide ou à une base pharmaceutiquement acceptables, seul ou en combinaison avec un ou plusieurs excipients pharmaceutiquement acceptables.

25. Compositions pharmaceutiques selon la revendication 24 utiles pour la fabrication d'un médicament pour le traitement et/ou la prophylaxie des hyperglycémies, des dyslipidémies, et plus particulièrement dans le traitement des diabètes non insulino-dépendants de type II, de la résistance à l'insuline, de l'intolérance au glucose, des désordres reliés au syndrome X, des maladies artérielles coronaires et d'autres maladies cardiovasculaires, des maladies rénales, des rétinopathies, des désordres reliés à l'activation des cellules endothéliales, du psoriasis, du syndrome polycystique ovarien, de la démence, de l'ostéoporose, des maladies inflammatoires intestinales, des dystrophies myotoniques, des pancréatites, de l'artériosclérose, du xanthome, mais également dans le traitement ou la prévention du diabète de type I, de l'obésité, de la régulation de l'appétit, de l'anorexie, de la boulimie, de l'anorexie nerveuse, ainsi que des pathologies cancéreuses et notamment les cancers hormono-dépendants tels que le cancer du sein et le cancer du colon, et en tant qu'inhibiteurs d'angiogénèse.

## Patentansprüche

1. Verbindungen der Formel (I): 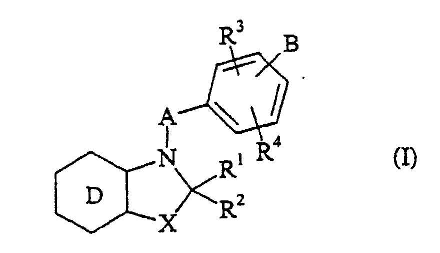 in der:
• X ein Sauerstoffatom, ein Schwefelatom oder eine Gruppe CH₂ oder 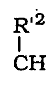 (worin R'² zusammen mit R² eine zusätzliche Bindung bildet) bedeutet,
• R¹ und R², die gleichartig oder verschieden sind, ein Wasserstoffatom, eine geradkettige oder verzweigte (C₁-C₆)-Alkylgruppe, Arylgruppe, geradkettige oder verzweigte Aryl-(C₁-C₆)-alkylgruppe, Aryloxygruppe, geradkettige oder verzweigte Aryl-(C₁-C₆)-alkyloxygruppe, geradkettige oder verzweigte (C₁-C₆)-Alkoxygruppe, Hydroxygruppe, Aminogruppe, geradkettige oder verzweigte (C₁-C₆)-Alkylaminogruppe oder geradkettige oder verzweigte (C₁-C₆)-Dialkylaminogruppe bedeuten,
oder R¹ und R² gemeinsam eine Oxo-, Thioxo- oder Iminogruppe bilden,
worin R² auch mit R'² eine zusätzliche Bindung bilden kann,
• A eine (C₁-C₆)-Alkylenkette bedeutet, bei der eine Gruppe CH₂ durch ein Heteroatom ausgewählt aus Sauerstoff oder Schwefel oder durch eine Gruppe NRₐ (worin Rₐ ein Wasserstoffatom oder eine geradkettige oder verzweigte (C₁-C₆)-Alkylgruppe darstellt) oder durch eine Phenylen- oder Naphthylengruppe ersetzt sein kann,
• B eine geradkettige oder verzweigte (C₁-C₆)-Alkylgruppe oder geradkettige oder, verzweigte (C₂-C₆)-Alkenylgruppe bedeutet, wobei diese Gruppen durch eine Gruppe R⁵, durch eine Gruppe der Formel (II): 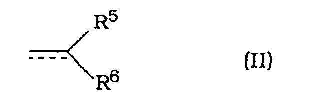 oder durch eine Gruppe der Formel (III): 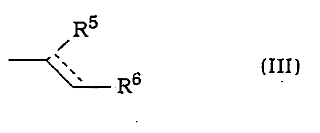 in denen:
- das Symbol ---- bedeutet, daß die Bindung einfach oder doppelt ist,
- R⁵ eine Gruppe 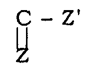 darstellt, in der Z ein Schwefelatom oder ein Sauerstoffatom und Z' eine Gruppe OR oder NRR' darstellen,
- und R⁶ eine Gruppe 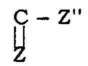 bedeutet, in der Z" eine Gruppe Z' oder R darstellt,
(oder R und R', die gleichartig oder verschieden sind, eine Gruppe R" oder -C(Me)₂COOR" bedeuten, worin R" ein Wasserstoffatom oder eine geradkettige oder verzweigte (C₁-C₆)-Alkylgruppe, geradkettige oder verzweigte (C₂-C₆)-Alkenylgruppe, geradkettige oder verzweigte (C₂-C₆)-Alkinylgruppe, Arylgruppe, geradkettige oder verzweigte Aryl-((C₁-C₆)-alkylgruppe, geradkettige oder verzweigte Aryl-(C₂-C₆)-alkenylgruppe, geradkettige oder verzweigte Aryl-(C₂-C₆)-alkinylgruppe, Heteroarylgruppe, geradkettige oder verzweigte Heteroaryl-(C₁-C₆)-alkylgruppe, geradkettige oder verzweigte Heteroaryl-(C₂-C₆)-alkenylgruppe, geradkettige oder verzweigte Heteroaryl-(C₂-C₆) -alkinylgruppe, (C₃-C₈)-Cycloalkylgruppe, geradkettige oder verzweigte (C3-C₈-Cycloalkyl-(C₁-C₆)-alkylgruppe oder geradkettige oder verzweigte Polyhalogen-(C₁-C₆)-alkyl-gruppe bedeutet) substituiert sind,
• R³ und R⁴, die gleichartig oder verschieden sind, ein Wasserstoffatom, ein Halogenatom oder eine Gruppe R, OR oder NRR' (worin R und R' die oben angegebenen Bedeutungen besitzen) bedeuten, oder R³ und R⁴ gemeinsam mit den sie tragenden Kohlenstoffatomen, wenn sie von zwei benachbarten Kohlenstoffatomen getragen werden, einen Ring mit 5 oder 6 Kettengliedern bilden, der ein Heteroatom ausgewählt aus Sauerstoff, Schwefel und Stickstoff enthalten kann,
• D:
einen Benzolkern darstellt und in diesem Fall X nicht eine Gruppe 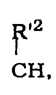 wie sie oben definiert worden ist, bedeutet,
oder D einen Pyridin-, Pyrazin-, Pyrimidin- oder Pyridazin-Kern darstellt, wobei diese Kerne nicht substituiert sind oder durch 1 bis 3 gleichartige oder verschiedene Gruppen ausgewählt aus R, OR, S(O)ₙR, C(Z)R, 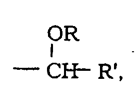 C(Z)OR, NRR', C(Z)NRR', 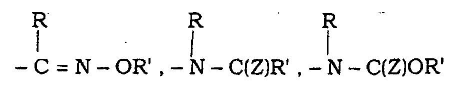 (worin R, R' und Z die oben angegebenen Bedeutungen besitzen und n 0, 1 oder 2 darstellt), Cyano, Nitro oder Halogenatome substituiert sind,
mit der Maßgabe, daß:
* wenn A eine Gruppe CH₂ darstellt, B nicht eine durch eine Gruppe 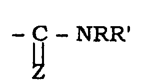 substituierte, geradkettige oder verzweigte (C₁-C₆₎-Alkylgruppe bedeutet,
* wenn die Gruppen A und B in ortho-Stellung zueinander an dem sie tragenden Benzolkern stehen, B nicht eine durch eine Gruppe 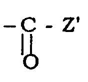 substituierte geradkettige oder verzweigte (C₂-C₆)-Alkenylengruppe bedeutet,
* wenn A eine Gruppe 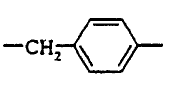 darstellt, B nicht eine Gruppe -CH₂-COOH bedeutet,
* unter Aryl eine Phenyl-, Naphthyl- oder Biphenylgruppe zu verstehen ist, wobei diese Gruppen teilweise hydriert sein können,
* unter Heteroaryl jede mono- oder bicyclische aromatische Gruppe zu verstehen ist, die 5 bis 10 Kettenglieder enthält und teilweise an den Ringen im Fall der bicyclischen Heteroarylgruppen hydriert sein kann und 1 bis 3 Heteroatome ausgewählt aus Sauerstoff, Stickstoff und Schwefel enthält,
wobei die in dieser Weise definierten Aryl- und Heteroarylgruppen durch 1 bis 3 Gruppen ausgewählt aus geradkettigem oder verzweigtem (C₁-C₆)-Alkyl, geradkettigem oder verzweigtem (C₁-C₆)-Alkoxy, Carboxy, Formyl, NR_{b}R_{c} (worin R_{b} und R_{c}, die gleichartig oder verschieden sind, Wasserstoffatome, geradkettige oder verzweigte (C₁-C₆)-Alkylgruppen, Arylgruppen oder Heteroarylgruppen darstellen), Estergruppen, Amidogruppen, Nitrogruppen, Cyanogruppen, Gruppen O-C(Me)₂COOR" (worin R" die oben angegebenen Bedeutungen besitzt) oder Halogenatome substituiert sein können,
deren Enantiomere und Diastereoisomere sowie deren Additionssalze mit einer pharmazeutisch annehmbaren Säure oder Base.

2. Verbindungen der Formel (I) nach Anspruch 1, worin R¹ und R² eine Oxogruppe bilden, deren Enantiomere und Diastereoisomere sowie deren Additionssalze mit einer pharmazeutisch annehmbaren Säure oder Base.

3. Verbindungen der Formel (I) nach Anspruch 1, worin R³ und R⁴ gleichzeitig Wasserstoffatome bedeuten, deren Enantiomere und Diastereoisomere sowie. deren Additionssalze mit einer pharmazeutisch annehmbaren Säure oder Base.

4. Verbindungen der Formel (I) nach Anspruch 1, worin A ene Alkylengruppe bedeutet, bei der eine CH₂-Gruppe durch ein Heteroatom ersetzt sein kann, deren Enantiomere und Diastereoisomere sowie deren Additionssalze mit einer pharmazeutisch annehmbaren Säure oder Base.

5. Verbindungen der Formel (I) nach Anspruch 1, worin A eine Ethylenoxygruppe bedeutet, deren Enantiomere und Diastereoisomere sowie deren Additionssalze mit einer pharmazeutisch annehmbaren Säure oder Base.

6. Verbindungen der Formel (I) nach Anspruch 1, worin A eine Alkylengruppe bedeutet, bei der eine CH₂-Gruppe durch eine Phenylen- oder Naphthylengruppe ersetzt sein kann, deren Enantiomere und Diastereoisomere sowie deren Additionssalze mit einer pharmazeutisch annehmbaren Säure oder Base.

7. Verbindungen der Formel (I) nach Anspruch 1, worin X ein Sauerstoff- oder Schwefelatom bedeutet, deren Enantiomere und Diastereoisomere sowie deren Additionssalze mit einer pharmazeutisch annehmbaren Säure oder Base.

8. Verbindungen der Formel (I) nach Anspruch 1, worin X eine Gruppe CHR'² und D einen Pyridinkern bedeuten, deren Enantiomere und Diastereoisomere sowie deren Additionssalze mit einer pharmazeutisch annehmbaren Säure oder Base.

9. Verbindungen der Formel (I) nach Anspruch 1, worin B eine Alkyl- oder Al-kenylgruppe bedeutet, wobei diese Gruppen durch eine Gruppe der Formel (II) substituiert sind, deren Enantiomere und Diastereoisomere sowie deren Additionssalze mit einer pharmazeutisch annehmbaren Säure oder Base.

10. Verbindungen der Formel (I) nach Anspruch 1, worin B eine Alkyl- oder Alkenylgruppe bedeutet, wobei diese Gruppen durch eine Gruppe R⁵ substituiert sind, deren Enantiomere und Diastereoisomere sowie deren Additionssalze mit einer pharmazeutisch annehmbaren Säure oder Base.

11. Verbindungen der Formel (I) nach Anspruch 1, worin D einen nichtsubstituierten Benzolkern bedeutet, deren Enantiomere und Diastereoisomere sowie deren Additionssalze mit einer pharmazeutisch annehmbaren Säure oder Base.

12. , Verbindungen der Formel (I) nach Anspruch 1, worin D einen Benzolkern bedeutet, der durch eine Arylcarbonylgruppe substituiert ist, deren Enantiomere und Diastereoisomere sowie deren Additionssalze mit einer pharmazeutisch annehmbaren Säure oder Base.

13. Verbndungen der Formel (I) nach Anspruch 1, worin D einen Pyridinkern bedeutet, deren Enantiomere und Diastereoisomere sowie deren Additionssalze mit einer pharmazeutisch annehmbaren Säure oder Base.

14. Verbindungen der Formel (I) nach Anspruch 1, worin D einen Pyrazin-, Pyrimidin- oder Pyridazinkern bedeutet, deren Enantiomere und Diastereoisomere sowie deren Additionssalze mit einer pharmazeutisch annehmbaren Säure oder Base.

15. Verbindungen der Formel (I) nach Anspruch 1, nämlich 2-{4-[-2-(2-Oxo-1,3-benzoxazol- 3(2*H*)-yl)-ethoxy]-benzyliden}-malonsäurediethylester, 2-{4-[2-(2-Oxo-1,3-benzothiazol-3(2*H)*-yl)-ethoxy]-benzyliden}-malonsäurediethylester und 2-{4-[3-(2-Oxo-1,3-benzothiazol-3(2*H*)-yl)-propoxyl-benzyliden}-malonsäurediethylester sowie deren Additionssalze mit einer pharmazeutisch annehmbaren Säure oder Base.

16. Verbindungen der Formel (I) nach Anspruch 1, nämlich 2-{4-[2-(2-Oxo-1,3-benzoxazol-3(2*H*)-yl)-ethoxy]-benzyl}-malonsäurediethylester, 3-Ethoxy-3-oxo-2-{4-[2-(2-oxo-1,3-benzoxazol-3(2*H*)-yl)-ethoxy]-benzyl}-propansäure, 2-{4-[2-(2-Oxo-1,3-benzoxazol-3(2*H*)-yl)-ethoxy]-benzyl}-malonsäure, 2-{4-[2-(2-Oxo-1,3-benzothiazol-3(2*H*)-yl)-ethoxy]-benzyl}-malonsäurediethylester, 2-{4-[2-(2-Oxo-1,3-benzothiazol-3(2*H*)-yl)-ethoxy]-benzyl}-malonsäuredimethylester, 3-Methoxy-3-oxo-2-{4=[2-(2=oxo-1,3-benzothiazol-3(2*H*)-yl)-ethoxy]-benzyl}-propansäure, 3-Ethoxy-3-oxo-2-{4-[2-(2-oxo-1,3-benzothiazol-3(2*H*)-yl)-ethoxy]-benzyl}-propansäure und 2-{4-[2-(2-Oxo-1,3-benzothiazol-3(2*H*)-yl)-ethoxy]-benzyl}-malonsäure sowie deren Additionssalze mit einer pharmazeutisch annehmbaren Säure oder Base.

17. Verbindungen der Formel (I) nach Anspruch 1, nämlich2-{4-[2-(6-Benzoyl-2-oxo-1,3-benzothiazol-3(2*H*)-yl)-ethoxy]-benzyl}-malonsäuredimethylester, 2-{4-[2-(6-Benzoyl-2-oxo-1,3-benzoxazol-3(2*H*)-yl)-ethoxy]-benzyl}-malonsäuredimethylester, 2-{4-[2-(6-(2-Chlorbenzoyl)-2-oxo-1,3-benzothiazol-3(2*H*)-yl)-ethoxy]-benzyl}malonsäuredimethylester, 2-{4-[2-(6-([1,1'-Biphenyl]-4-yl-carbonyl)-2-oxo-1,3-benzothiazol-3(2*H*)-yl)-ethoxyl]-benzyl}-malonsäuredimethylester, 2-(4-[2-(6-(1-Naphthoyl)-2-oxo-1,3-benzothiazol-3(2*H*)-yl)-ethoxy]-benzyl}-malonsäuredimethylester, 2-{4-[2-(6-(3-Chlorbenzoyl)-2-oxo-1,3-benzothiazol-3(2*H*)-yl-ethoxy]-benzyl}-malonsäuredimethylester, 2-{4-[2-(2-Oxo-6-(3-pyridinylcarbonyl)-1,3-benzothiazol-3(2*H*)-yl)-ethoxy]-benzyl}-malonsäuredimethylester, 2-{4-[2-(6-(4-Chlorbenzoyl)-2-oxo-1,3-benzothiazol-3(2*H*)-yl)-ethoxy] -benzyl}-malonsäuredimethylester, 2-{4-[2-(6-Benzoyl-2-oxo-1,3-benzothiazol-3(2*H*)-yl)-ethoxyl-benzyl}-malonsäure. 2-{4-[2-(6-(2-Naphthoyl)-2-oxo-1,3-benzothiazol-3(2*H*)-yl)-ethoxyl-benzyl}-malonsäuredimethylester, 2-{4-[2-(6-(4-Methoxybenzoyl)-2-oxo-1,3-benzothiazol-3(2*H*)-yl)-ethoxyl-benzyl}-malonsäüredimethylester, 2-{4-(2-(6-Benzoyl-2-oxo-1,3-benzothiazol-3(2*H*)-yl)-ethoxy]-benzyl}-3-methoxy-3-oxopropansäure und 2-{4-[2-(6-Benzoyl-2-oxo-1,3-benzothiazol-3(2*H*)-yl)-ethoxy]-benzyl}-3-(methylamino)-3-oxopropansäuremethylester sowie deren Additionssalze mit einer pharmazeutisch annehmbaren Säure oder Base.

18. Verbindungen der Formel (I) nach Anspruch 1, nämlich 2-{4-[2-(6-Benzyl-2-oxo-1,3-benzothiazol-3(2*H*)-yl)-ethoxy]-benzyl}-malonsäuredimethylester, 2-{4-[2-(6-Benzyl-2-oxo-1,3-benzoxazol-3(2*H*)-yl)-ethoxy]-benzyl}-malonsäuredimethylester, 2-{4-[2-(6-(2-Chlorbenzyl)-2-oxo-1,3-benzothiazol-3(2*H*)-yl)-ethoxy]-benzyl}malonsäuredimethylester, 2-[4-[2-(6-(3-Chlorbenzyl)-2-oxo-1,3-benzothiazol-3(2*H*)-yl)-ethoxy]-benzyl}-malonsäuredimethylester, 2-{4-[2-(6-(1-Naphthylmethyl)-2-oxo-1,3-benzothiazol-3(2*H*)-yl)-ethoxy]-benzyl}-malonsäuredimethylester und 2-{4-[2-(6-(4-Chlorbenzyl)-2-oxo-1,3-benzothiazol-3(2*H*)-yl)-ethoxy]-benzyl}-malon säuredimethylester sowie deren Additionssalze mit einer pharmazeutisch annehmbaren Säure oder Base.

19. Verbindungen der Formel (I) nach Anspruch 1, nämlich 2-{4-[2-(2-Oxo[1,3]-oxazolo[4,5-*b*]pyridin-3(2*H*)-yl)-ethoxy]-benzyl}-malonsäuredimethylester, 2-{4-[2-(1*H*-Pyrrolo[2,3-*b*]pyridin-1-yl)-ethoxy]-benzyl}-malonsäuredimethylester und 2-{4-[2-(1*H*-Pyrrolo[2,3-*b*]pyridin-1-yl)-ethoxy]-benzyl}-malonsäure sowie deren Additionssalze mit einer pharmazeutisch annehmbaren Säure oder Base.

20. Verbindungen der Formel (I) nach Anspruch 1, nämlich 2-{4-[2-(6-[Hydroxy-(phenyl)-methyl]-2-oxo-1,3-benzoxazol-3(2*H*)-yl)-ethoxy]-benzyl}-malonsäuredimethylester und 2-{4-[2-(6-[Hydroxy-(phenyl)-methyl]-2-oxo-1,3-benzothiazol-3(2*H*)-yl)-ethoxy]-benzyl}-malonsäuredimethylester sowie deren Additionssalze mit einer pharmazeutisch annehmbaren Säure oder Base.

21. Verbindungen der Formel (I) nach Anspruch 1, nämlich 2-{4-(2-(6-[(Methoxyimino)(phenyl)-methyl]-2-oxo-1,3-benzothiazol-3(2*H*)-yl)-ethoxy]-benzyl}-malonsäuredimethylester, 2-{4-[2-(6-[(Hydroxyimino)(phenyl)-methyl]-2-oxo-1,3-benzothiazol-3(2*H*)-yl)-ethoxy]-benzyl}-malonsäuredimethylester, 2-{4-[2-(6-[(3-Chlorphenyl)(methoxyimino)-methyl]-2-oxo-1,3-benzothiazol-3(2*H*)-yl)-ethoxy]-benzyl}-malonsäuredimethylester und 2-{4-[2-(6-[[1,1'-Biphenyl]-4-yl-(methoxyimino)-methyl]-2-oxo-1,3-benzothiazol-3(2*H*)-yl)-ethoxy]-benzyl}-malonsäuredimethylester sowie deren Additionssalze mit einer pharmazeutisch annehmbaren Säure oder Base.

22. Verbindungen der Formel (I) nach Anspruch 1, nämlich 3-{4-[3-(2-Oxo-1,3-benzoxazol-3(2*H*)-yl)-propyl]-phenyl}-2-propensäure, 3-{4-[3-(2-Oxo-1,3-benzoxazol-3(2*H*)-yl)-propyl]-phenyl}-2-propansäure, 3-{4-[3-(2-Oxo-1,3-benzothiazol-3(2*H*)-yl)-propyl]-phenyl}-2-propensäure, 3-{4-[3-(2-Oxo-1,3-benzothiazol-3(2*H*)-yl)-propyl]-phenyl}-2-propansäure, 3-{4-[2-(6-Benzoyl-2-oxo-1,3-benzothiazol-3(2*H*)-yl)-ethoxy]-phenyl}-propansäuremethylester, 3-{4-(2-(6-Benzoyl-2-oxo-1,3-benzoxazol-3 (2*H*)-yl)-ethoxy]-phenyl}-propansäuremethylester, 3-{4-[2-(6-Benzyl-2-oxo-1,3-benzothiazol-3(2*H*)-yl)-ethoxy]-phenyl}-propansäuremethylester, 3-{4-[2-(6-[(Methoxyimino)(phenyl)-methyl]-2-oxo-1,3-benzothiazol-3(2*H*)-yl)-ethoxy]-phenyl}-propansäuremethylester und 3-{4-[2-(6-Benzoyl-2-oxo-1,3-benzothiazol-3(2*H*)-yl)-ethoxy]-phenyl}-propansäure sowie deren Additionssalze mit einer pharmazeutisch annehmbaren Säure oder Base.

23. Verfahren zur Herstellung der Verbindungen der Formel (I) nach Anspruch 1, **dadurch gekennzeichnet, daß** man als Ausgangsprodukt eine Verbindung der Formel (IV) verwendet: 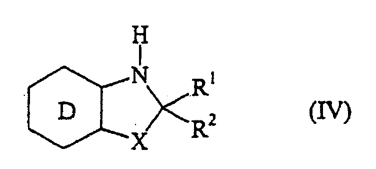 in der R¹, R², X und D die bezüglich der Formel (I) angegebenen Bedeutungen besitzen,
welche man in basischem Medium mit einer Verbindung der Formel (V) kondensiert: 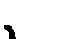 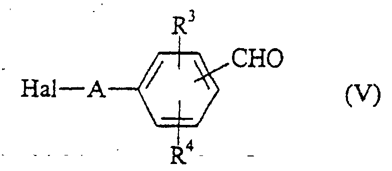 in der Hal ein Halogenatom bedeutet und A, R³ und R⁴ die bezüglich der Formel (I) angegebenen Bedeutungen besitzen,
zur Bildung der Verbindung der Formel (VI): 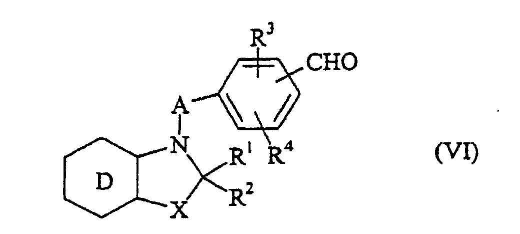 in der A, D, X, R¹, R², R³ und R⁴ die oben angegebenen Bedeutungen besitzen,
- welche man in basischem Medium mit einer Verbindung der Formel (VII) kondensiert: 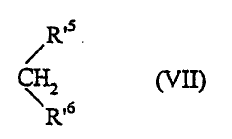 in der R'⁵ und R'⁶ die oben für R⁵ und R⁶ angegebenen Bedeutungen mit Ausnahme der Gruppe COOH besitzen,
zur Bildung der Verbindung der Formel (I/a), einem Sonderfall der Verbindungen der Formel (I): 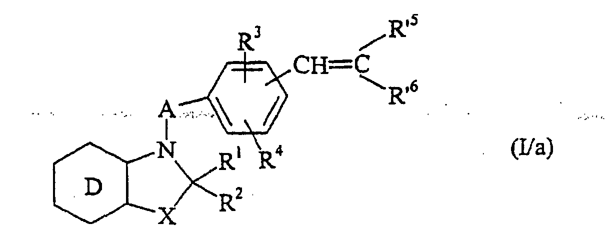 in der D, A, X, R¹, R², R³, R⁴, R'⁵ und R'⁶ die oben angegebenen Bedeutungen besitzen,
welche in Gegenwart eines Katalysators wie beispielweise Palladium-auf-Kohlenstoff, hydriert wird, so daß man die Verbindung der Formel (I/b) erhält, einem Sonderfall der Verbindungen der Formel (I): 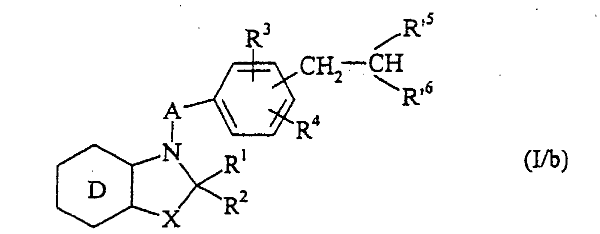 in der D, A, X, R¹, R², R³, R⁴, R^{'5} und R'⁶ die oben angegebenen Bedeutungen besitzen,
(wobei die Verbindungen der Formeln (I/a) und (I/b) auch durch direkte Kondensation der Verbindung der Formel (IV) mit einer Verbindung der Formel (V') erhalten werden können: 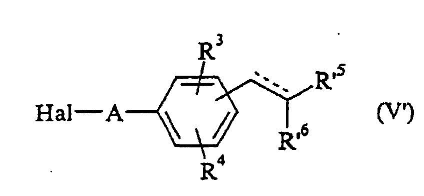 in der Hal ein Halogenatom darstellt und A, R³, R⁴, R'⁵ und R'⁶ die oben angegebenen Bedeutungen besitzen),
- oder man die Verbindung der Formel (VI) unter den Bedingungen einer Horner-Emmons-Reaktion mit der entsprechenden Phosphonverbindung eines Derivats der Formel (VIII) kondensiert:
Hal - CH₂ - B' (VIII)
in der Hal ein Halogenatom bedeutet und B' eine geradkettige oder verzweigte (C₁-C₅)-Alkylgruppe oder geradkettige oder verzweigte (C₂-C₅)-Alkenylgruppe darstellt, wobei diese Gruppen durch eine Gruppe R'⁵ oder durch eine Gruppe der Formel (II') 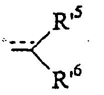 oder durch eine Gruppe der Formel (III') 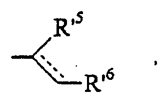 worin R'⁵ und R'⁶ und das Symbol ---- die oben angegebenen Bedeutungen besitzen, substituiert sind,
zur Bildung der Verbindung der Formel (I/c), einem Sonderfall der Verbindungen der Formel (I): 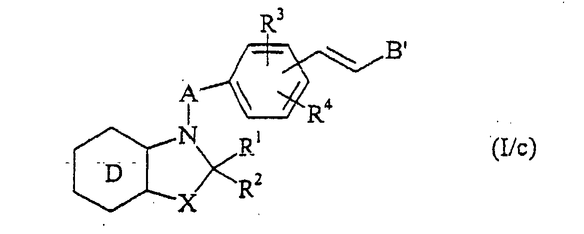 in der D, X, A, B', R¹, R², R³ und R⁴ die oben angegebenen Bedeutungen besitzen,
welche einer katalytischen Reaktion, beispielsweise in Gegenwart von Palladium, unterworfen werden kann zur Bildung der Verbindung der Formel (I/d), nem Sonderfall der Verbindungen der Formel (I): 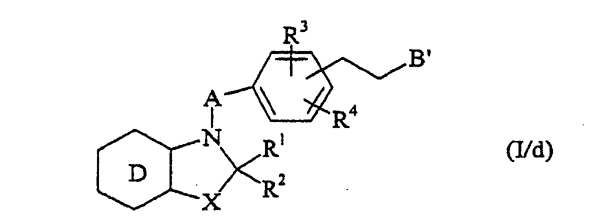 in der D, A, X, B', R¹, R², R³ und R⁴ die oben angegebenen Bedeutungen besitzen,
- oder man die Verbindung der Formel (VI) in das entsprechende Säurechlorid umwandelt, welches man, beispielsweise in Gegenwart eines Palladium- oder Zinn-Derivats, mit der Verbindung der Formel (IX) kondensiert: 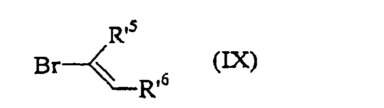 in der R'⁵ und R'⁶ die oben angegebenen Bedeutungen besitzen, zur Bildung der Verbindung der Formel (X): 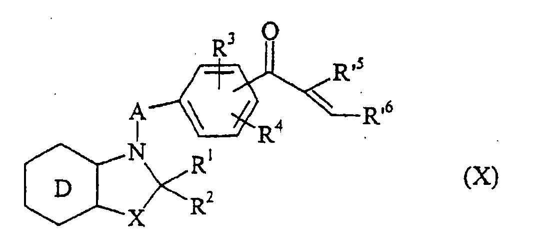 in der D, X, A, R¹, R², R³, R⁴, R'⁵ und R'⁶ die oben angegebenen Bedeutungen besitzen,
welche man der Einwirkung eines Reduktionsmittels, wie beispielsweise Et₃SiH, unterwirft zur Bildung der Verbindung der Formel (I/e), einem Sonderfall der Verbindungen der Formel (I): 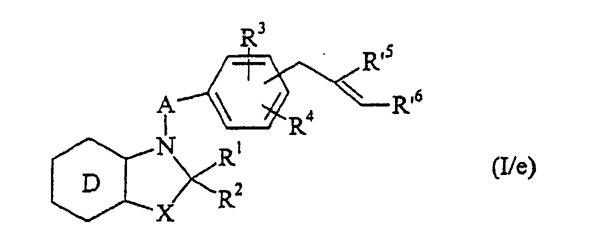 in der D, X, A, R¹, R², R³, R⁴, R'⁵ und R'⁶ die oben angegebenen Bedeutungen besitzen,
welche Verbindungen der Formeln (I/a), (I/b), (I/c), (I/d) und (I/e), worin R'⁵ und R'⁶ Estergruppen bedeuten, vollständig oder teilweise verseift werden können, so daß man die entsprechenden gem-dicarboxylierten oder hemi-carboxylierten Verbindungen der Formel (I/f) erhält, einem Sonderfall der Verbindungen der Formel (I): 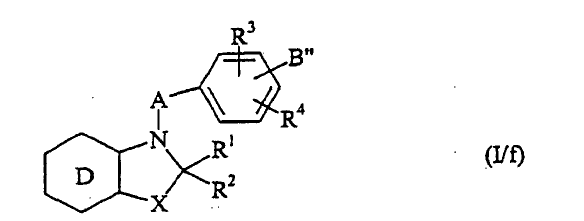 in der D, X, A, R¹, R², R³ und R⁴ die oben angegebenen Bedeutungen besitzen und B" eine Gruppe, wie sie oben für B definiert worden ist, darstellt,
worin R⁵ und/oder R⁶ eine Gruppe COOH bedeuten,
(wobei die Verbindungen der Formel (I/f), worin B" eine Gruppe -CH=CH-COOH darstellt, direkt erhalten werden können ausgehend von der Verbindung der Formel (VI) durch Kondensation mit Malonsäure unter decarboxylierenden Bedingungen, und welche reduziert werden können zur Bildung der Verbindungen der Formel (I/f), worin B" eine Gruppe -CH₂-CH₂-COOH darstellt),
wobei die Verbindungen der Formeln (I/a) bis (I/f), die die Gesamtheit der erfindungsgemäßen Verbindungen darstellen, gemäß einer klassischen Trennmethode gereinigt werden können, welche gewünschtenfalls in ihre Additionssalze mit einer pharmazeutisch annehmbaren Säure oder Base umgewandelt werden können und die gegebenenfalls mit Hilfe einer klassischen Trennmethode in ihre Isomeren aufgetrennt werden können.

24. Pharmazeutische Zubereitungen, enthaltend als Wirkstoff mindestens eine Verbindung der Formel (I) nach einem der Ansprüche 1 bis 22 oder eines ihrer Additionssalze mit einer pharmazeutisch annehmbaren Säure oder Base allein oder in Kombination mit einem oder mehreren pharmazeutisch annehmbaren Trägermaterialien.

25. Pharmazeutische Zubereitungen nach Anspruch 24 nützlich für die Herstellung eines Arzneimittels zur Behandlung und/oder der Prophylaxe von Hyperglykämien, Dyslipidämien und insbesondere bei der Behandlung des nicht insulinabhängigen Diabetes des Typs II, der Insulinresistenz, der Glucoseunverträglichkeit, von Störungen, die mit dem X-Syndrom verknüpft sind, von Koronararterienkrankheiten und anderen kardiovaskulären Erkrankungen, Nierenkrankheiten, Retinopathien, Störungen, die mit der Aktivierung von Endothelialzellen verknüpft sind, von Psoriasis, dem polyzystischen Ovarialsyndrom, der Demenz, der Osteoporose, von Darmentzündungen, myotonischen Dystrophien, Pankreatitis, Arteriosklerose, Xanthomen, jedoch auch zur Behandlung oder Prophylaxe des Diabetes des Typs I, der Fettsucht, für die Steuerung der Appetits, der Appetitlosigkeit, der Bulimie, der nervösen Appetitlosigkeit sowie zur Behandlung von krebsartigen pathologischen Zuständen und insbesondere Hormon-abhängigen Krebsen, wie Brustkrebs und Darmkrebs sowie als Inhibitoren der Angiogenese.

## Claims

1. Compounds of formula (I) : 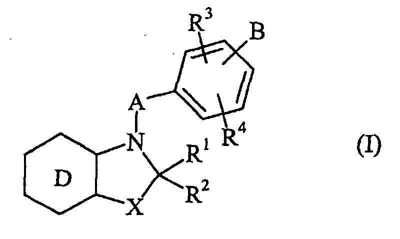 wherein:
• X represents an oxygen or sulphur atom or a CH₂ or 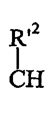 group (wherein R'² together with R² forms an additional bond),
• R¹ and R², which may be identical or different, each represents a hydrogen atom, a linear or branched (C₁-C₆)alkyl group, an aryl group, an aryl-(C₁-C₆)alkyl group in which the alkyl moiety may be linear or branched, an aryloxy group, an aryl-(C₁-C₆)alkoxy group in which the alkoxy moiety may be linear or branched, a linear or branched (C₁-C₆)alkoxy group, a hydroxy group, an amino group, a linear or branched (C₁-C₆)alkylamino group or a di-(C₁-C₆)alkylamino group in which the alkyl moieties may be linear or branched,
or R¹ and R² together form an oxo, thioxo or imino group,
it being possible furthermore for R² to form with R'² an additional bond,
• A represents a (C₁-C₆)alkylene chain in which a CH₂ group may be replaced by a hetero atom selected from oxygen and sulphur, by an NRₐ group (wherein Rₐ represents a hydrogen atom or a linear or branched (C₁-C₆)alkyl group), or by a phenylene or naphthylene group,
• B represents a linear or branched (C₁-C₆)alkyl group or a linear or branched (C₂-C₆)alkenyl group, those groups being substituted by an R⁵ group, by a group of formula (II) : 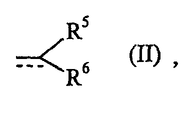 or by a group of formula (III) 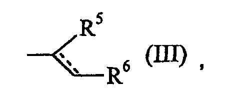 in which groups:
- the representation ---- denotes that the bond is single or double,
- R⁵ represents a 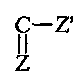 group wherein Z represents a sulphur atom or an oxygen atom and Z' represents an OR or NRR' group,
- and R⁶ represents a group 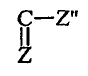 wherein Z" represents a Z' or R group,
(wherein R and R', which may be identical or different, each represents an R" or -C(Me)₂COOR" group wherein R" represents a hydrogen atom or a linear or branched (C₁-C₆)alkyl group, a linear or branched (C₂-C₆)alkenyl group, a linear or branched (C₂-C₆)alkynyl group, an aryl group, an aryl-(C₁-C₆)alkyl group in which the alkyl moiety may be linear or branched, an aryl-(C₂-C₆)alkenyl group in which the alkenyl moiety may be linear or branched, an aryl-(C₂-C₆)alkynyl group in which the alkynyl moiety may be linear or branched, a heteroaryl group, a heteroaryl-(C₁-C₆)alkyl group in which the alkyl moiety may be linear or branched, a heteroaryl-(C₂-C₆)alkenyl group in which the alkenyl moiety may be linear or branched, a heteroaryl-(C₂-C₆)alkynyl group in which the alkynyl moiety may be linear or branched, a (C₃-C₈)cycloalkyl group, a (C₃-C₈)cycloalkyl-(C₁-C₆)alkyl group in which the alkyl moiety may be linear or branched, or a linear or branched (C₁-C₆)polyhaloalkyl group),
• R³ and R⁴, which may be identical or different, each represents a hydrogen atom, a halogen atom or an R, OR or NRR' group (wherein R and R' are as defined hereinbefore),
or R³ and R⁴ together with the carbon atoms carrying them, when they are carried by two adjacent carbon atoms, form a ring that has 5 or 6 ring members and that may contain a hetero atom selected from oxygen, sulphur and nitrogen,
• D represents :
a benzene nucleus, in which case X cannot represent a group 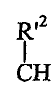 as defined hereinbefore,
or D represents a pyridine, pyrazine, pyrimidine or pyridazine nucleus,
those nuclei being unsubstituted or substituted by from 1 to 3 identical or different groups selected from R, OR, S(O)ₙR, C(Z)R, 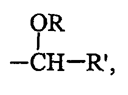 C(Z)OR, NRR', C(Z)NRR', 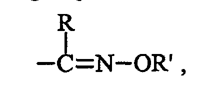 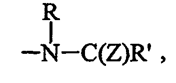 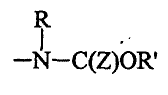 (in which groups R, R' and Z are as defined hereinbefore and n is 0, 1 or 2), cyano, nitro and halogen atoms,
wherein:
* when A represents a CH₂ group, B cannot represent a linear or branched (C₁-C₆)alkyl group substituted by a group 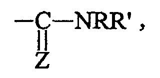
* when the groups A and B are in the ortho position in relation to one another on the benzene nucleus carrying them, B cannot represent a linear or branched (C₂-C₆)-alkenylene group substituted by a group 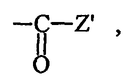
* when A represents a group 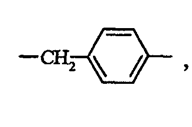 B cannot represent a -CH₂-COOH group,
* aryl is to be understood as a phenyl, naphthyl or biphenyl group, which groups may be partially hydrogenated,
* heteroaryl is to be understood as any mono- or bi-cyclic aromatic group containing from 5 to 10 ring members, which may be partially hydrogenated on one of the rings in the case of bicyclic heteroaryls, and which contains from 1 to 3 hetero atoms selected from oxygen, nitrogen and sulphur,
wherein the aryl and heteroaryl groups so defined may be substituted by from 1 to 3 groups selected from linear or branched (C₁ -C₆)alkyl, linear or branched (C₁-C₆)alkoxy, carboxy, formyl, NR_{b}R_{c} (wherein R_{b} and R_{c}, which may be identical or different, each represents a hydrogen atom, a linear or branched (C₁-C₆)alkyl group, an aryl group or a heteroaryl group), ester, amido, nitro, cyano, O-C(Me)₂COOR" (wherein R" is as defined hereinbefore) and halogen atoms,
their enantiomers and diastereoisomers, and also pharmaceutically acceptable addition salts thereof with an acid or a base.

2. Compounds of formula (I) according to claim 1, wherein R¹ and R² form an oxo group, their enantiomers and diastereoisomers, and also pharmaceutically acceptable addition salts thereof with an acid or a base.

3. Compounds of formula (I) according to claim 1, wherein R³ and R⁴ simultaneously represent a hydrogen atom, their enantiomers and diastereoisomers, and also pharmaceutically acceptable addition salts thereof with an acid or a base.

4. Compounds of formula (I) according to claim 1, wherein A represents an alkylene group in which a CH₂ group may be replaced by a hetero atom, their enantiomers and diastereoisomers, and also pharmaceutically acceptable addition salts thereof with an acid or a base.

5. Compounds of formula (I) according to claim 1, wherein A represents an ethyleneoxy group, their enantiomers and diastereoisomers, and also pharmaceutically acceptable addition salts thereof with an acid or a base.

6. Compounds of formula (I) according to claim 1, wherein A represents an alkylene group in which a CH₂ group may be replaced by a phenylene or naphthylene group, their enantiomers and diastereoisomers, and also pharmaceutically acceptable addition salts thereof with an acid or a base.

7. Compounds of formula (I) according to claim 1, wherein X represents an oxygen atom or a sulphur atom, their enantiomers and diastereoisomers, and also pharmaceutically acceptable addition salts thereof with an acid or a base.

8. Compounds of formula (I) according to claim 1, wherein X represents a CHR'² group and D represents a pyridine nucleus, their enantiomers and diastereoisomers, and also pharmaceutically acceptable addition salts thereof with an acid or a base.

9. Compounds of formula (I) according to claim 1, wherein B represents an alkyl or alkenyl group, those groups being substituted by a group of formula (II), their enantiomers and diastereoisomers, and also pharmaceutically acceptable addition salts thereof with an acid or a base.

10. Compounds of formula (I) according to claim 1, wherein B represents an alkyl or alkenyl group, those groups being substituted by an R⁵ group, their enantiomers and diastereoisomers, and also pharmaceutically acceptable addition salts thereof with an acid or a base.

11. Compounds of formula (I) according to claim 1, wherein D represents an unsubstituted benzene nucleus, their enantiomers and diastereoisomers, and also pharmaceutically acceptable addition salts thereof with an acid or a base.

12. Compounds of formula (I) according to claim 1, wherein D represents a benzene nucleus substituted by an arylcarbonyl group, their enantiomers and diastereoisomers, and also pharmaceutically acceptable addition salts thereof with an acid or a base.

13. Compounds of formula (I) according to claim 1, wherein D represents a pyridine nucleus, their enantiomers and diastereoisomers, and also pharmaceutically acceptable addition salts thereof with an acid or a base.

14. Compounds of formula (I) according to claim 1, wherein D represents a pyrazine, pyrimidine or pyridazine nucleus, their enantiomers and diastereoisomers, and also pharmaceutically acceptable addition salts thereof with an acid or a base.

15. Compounds of formula (I) according to claim 1, which are diethyl 2-{4-[2-(2-oxo-1,3-benzoxazol-3(2*H*)-yl)ethoxy]benzylidene}malonate, diethyl 2-{4-[2-(2-oxo-1,3-benzothiazol-3(2*H*)-yl)ethoxy]benzylidene}malonate and diethyl 2-{4-[3-(2-oxo-1,3-benzothiazol-3(2*H*)-yl)propoxy]benzylidene}malonate, and also pharmaceutically acceptable addition salts thereof with an acid or a base.

16. Compounds of formula (I) according to claim 1, which are diethyl 2-{4-[2-(2-oxo-1,3-benzoxazol-3(2*H*)-yl)ethoxy]benzyl) malonate, 3-ethoxy-3-oxo-2-{4-[2-(2-oxo-1,3-benzoxazol-3(2*H*)-yl)ethoxy]benzyl)propanoic acid, 2-{4-[2-(2-oxo-1,3-benzoxazol-3(2*H*)-yl)ethoxy]benzyl}malonic acid, diethyl 2-{4-[2-(2-oxo-1,3-benzothiazol-3(2*H*)-yl)ethoxy]benzyl)malonate, dimethyl 2-{4-[2-(2-oxo-1,3-benzothiazol-3(2*H*)-yl)-ethoxy]benzyl}malonate, 3-methoxy-3-oxo-2-{4-[2-(2-oxo-1,3-benzothiazol-3(2*H*)-yl)ethoxy]benzyl}propanoic acid, 3-ethoxy-3-oxo-2-{4-[2-(2-oxo-1,3-benzothiazol-3(2*H*)-yl)ethoxy]benzyl}propanoic acid and 2-{4-[2-(2-oxo-1,3-benzothiazol-3(2*H*)-yl)ethoxy]benzyl}malonic acid, and also pharmaceutically acceptable addition salts thereof with an acid or a base.

17. Compounds of formula (I) according to claim 1, which are dimethyl 2-{4-[2-(6-benzoyl-2-oxo-1,3-benzothiazol-3(2*H*)-yl)ethoxy]benzyl}malonate, dimethyl 2-{4-[2-(6-benzoyl-2-oxo-1,3-benzoxazol-3(2*H*)-yl)ethoxy]benzyl}malonate, dimethyl 2-{4-[2-(6-(2-chlorobenzoyl}-2-oxo-1,3-benzothiazol-3(2*H*)-yl)ethoxy]benzyl}malonate, dimethyl 2-{4-[2-(6-([1,1'-biphenyl]-4-ylcarbonyl)-2-oxo-1,3-benzothiazol-3(2*H*)-yl)ethoxy]-benzyl}malonate, dimethyl 2-{4[2-(6(1-naphthoyl)-2-oxo-1,3-benzothiazol-3(2*H*)-yl)ethoxy]benzyl}malonate, dimethyl 2-{4-[2-(6-(3-chlorobenzoyl)-2-oxo-1,3-benzothiazol-3(2*H*)-yl)ethoxy]benzyl}malonate, dimethyl 2-{4-[2-(2-oxo-6-(3-pyridylcarbonyl)-1,3-benzothiazol-3(2*H*)-yl)ethoxy]benzyl}malonate, dimethyl 2-{4-[2-(6-(4-chlorobenzoyl)-2-oxo-1,3-benzothiazol-3(2*H*)-yl)ethoxy]benzyl}malonate, 2-{4-[2-(6-benzoyl-2-oxo-1,3-benzothiazol-3(2*H*)-yl)ethoxy]benzyl}malonic acid, dimethyl 2-{4-[2-(6-(2-naphthoyl)-2-oxo-1,3-benzothiazol-3(2*H*)-yl)ethoxy]benzyl}malonate, dimethyl 2-{4-[2-(6-(4-methoxybenzoyl)-2-oxo-1,3-benzothiazol-3(2*H*)-yl)ethoxy]-benzyl}malonate, 2-{4-[2-(6-benzoyl-2-oxo-1,3-benzothiazol-3(2*H*)-yl)ethoxy]benzyl}-3-methoxy-3-oxopropanoic acid and methyl 2-{4-[2-(6-benzoyl-2-oxo-1,3-benzothiazol-3(2*H*)-yl)ethoxy]benzyl}-3-(methylamino)-3-oxopropanoate, and also pharmaceutically acceptable addition salts thereof with an acid or a base.

18. Compounds of formula (I) according to claim 1, which are dimethyl 2-{4-[2-(6-benzyl-2-oxo-1,3-benzothiazol-3(2*H*)-yl)ethoxy]benzyl}malonate, dimethyl 2-{4-[2-(6-benzyl-2-oxo-1,3-benzoxazol-3(2*H*)-yl)ethoxy]benzyl}malonate, dimethyl 2-{4-[2-(6-(2-chlorobenzyl)-2-oxo-1,3-benzothiazol-3(2*H*)-yl)ethoxy]benzyl}malonate, dimethyl 2-{4-[2-(6-(3-chlorobenzyl)-2-oxo-1,3-benzothiazol-3(2*H*)-yl)ethoxy]-benzyl}malonate, dimethyl 2-{4-[2-(6-(1-naphthylmethyl)-2-oxo-1,3-benzothiazol-3(2*H*)-yl)ethoxy]benzyl}malonate and dimethyl methyl 2-(4-[2-(6-(4-chlorobenzyl)-2-oxo-1,3-benzothiazol-3(2*H*)-yl)ethoxy]benzyl}malonate, and also pharmaceutically acceptable addition salts thereof with an acid or a base.

19. Compounds of formula (I) according to claim 1, which are dimethyl 2-{4-[2-(2-oxo[1,3]oxazolo[4,5-*b*]pyrid-3(2*H*)-yl)ethoxy]benzyl}malonate, dimethyl 2-{4-[2-(1*H*-pyrrolo[2,3-*b*]pyrid-1-yl)ethoxy]benzyl}malonate and 2-{4-[2-(1*H*-pyrrolo[2,3-*b*]pyrid-1-yl)ethoxy]benzyl}malonic acid, and also pharmaceutically acceptable addition salts thereof with an acid or a base.

20. Compounds of formula (I) according to claim 1, which are dimethyl 2-{4-[2-(6-[hydroxy(phenyl)methyl]-2-oxo-1,3-benzoxazol-3(2*H*)-yl)ethoxy]benzyl}malonate and dimethyl 2-{4-[2-(6-[hydroxy(phenyl)methyl]-2-oxo-1,3-benzothiazol-3(2*H*)-yl)ethoxy]benzyl}malonate, and also pharmaceutically acceptable addition salts thereof with an acid or a base.

21. Compounds of formula (I) according to claim 1, which are dimethyl 2-{4-[2-(6-[(methoxyimino)(phenyl)methyl]-2-oxo-1,3-benzothiazol-3(2*H*)-yl)ethoxyl]benzyl}-malonate, dimethyl 2-{4-[2-(6-[(hydroxyimino)(phenyl)methyl]-2-oxo-1,3-benzothiazol-3(2*H*)-yl)ethoxy]benzyl}malonate, dimethyl 2-{4-[2-(6-[(3-chlorophenyl)-(methoxyimino)methyl]-2-oxo-1,3-benzothiazol-3(2*H*)-yl)ethoxy]benzyl}malonate and dimethyl 2-{4-[2-(6-[[1,1'-biphenyl]-4-yl(methoxyimino)methyl]-2-oxo-1,3-benzothiazol-3(2*H*)-yl)ethoxy]benzyl}malonate, and also pharmaceutically acceptable addition salts thereof with an acid or a base.

22. Compounds of formula (I) according to claim 1, which are 3-{4-[3-(2-oxo-1,3-benzoxazol-3(2*H*)-yl)propyl]phenyl} -2-propenoic acid, 3-{4-[3-(2-oxo-1,3-benzoxazol-3(2*H*)-yl)propyl]phenyl}-2-propanoic acid, 3-{4-[3-(2-oxo-1,3-benzothiazol-3(2*H*)-yl)propyl]phenyl}-2-propenoic acid, 3-{4-[3-(2-oxo-1,3-benzothiazol-3(2*H*)-yl)-propyl]phenyl}-2-propanoic acid, methyl 3-{4-[2-(6-benzoyl-2-oxo-1,3-benzothiazol-3(2*H*)-yl)ethoxy]phenyl}propanoate, methyl 3-{4-[2-(6-benzoyl-2-oxo-1,3-benzoxazol-3(2*H*)-yl)ethoxy]phenyl}propanoate, methyl 3-{4-[2-(6-benzyl-2-oxo-1,3-benzothiazol-3(2*H*)-yl)ethoxy]phenyl}propanoate, methyl 3-{4-[2-(6-[(methoxyimino)(phenyl)methyl]-2-oxo-1,3-benzothiazol-3(2*H*)-yl)ethoxy]phenyl}propanoate and 3-{4-[2-(6-benzoyl-2-oxo-1,3-benzothiazol-3(2*H*)-yl}ethoxy]phenyl}propanoic acid, and also pharmaceutically acceptable addition salts thereof with an acid or a base.

23. Process for the preparation of compounds of formula (I) according to claim 1, **characterised in that** there is used as starting material a compound of formula (IV): 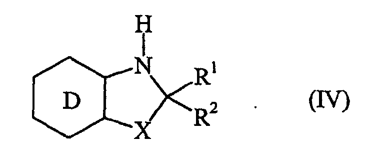 wherein R¹, R², X and D are as defined for formula (I)_{,}
which is condensed in basic medium with a compound of formula (V) : 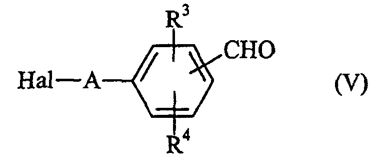 wherein Hal represents a halogen atom and A, R³ and R⁴ are as defined for formula (I),
to yield a compound of formula (VI): 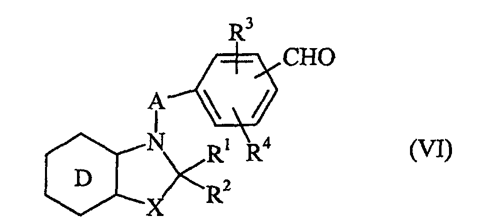 wherein A, D, X, R¹, R², R³ and R⁴ are as defined hereinbefore,
- which is condensed in basic medium with a compound of formula (VII): 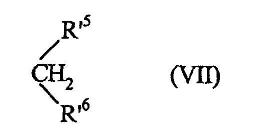
wherein R'⁵ and R'⁶ may have any of the meanings given hereinbefore for R⁵ and R⁶ with the exception of the group COOH, to obtain a compound of formula (I/a), a particular case of the compounds of formula (I): 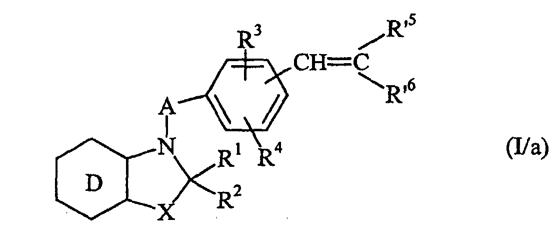 wherein D, A, X, R¹, R², R³, R⁴, R'⁵ and R'⁶ are as defined hereinbefore,
which is hydrogenated in the presence of a catalyst, such as palladium-on-carbon for example, to yield a compound of formula (I/b), a particular case of the compounds of formula (I): 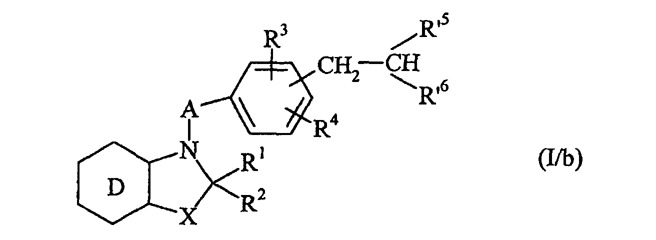 wherein D, A, X, R¹, R², R³, R⁴, R^{'5} and R'⁶ are as defined hereinbefore,
(it being possible for the compounds of formulae (I/a) and (I/b) to be obtained by direct condensation of a compound of formula (IV) with a compound of formula (V'): 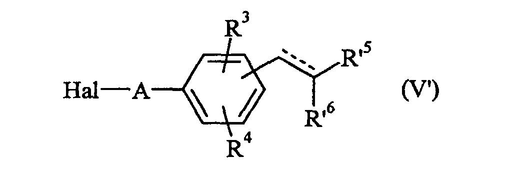 wherein Hal represents a halogen atom and A, R³, R⁴, R'⁵ and R'⁶ are as defined hereinbefore),
- or which compound of formula (VI) is condensed, under the conditions of a Horner-Emmons reaction, with a corresponding phosphonic compound of a compound of formula (VIII)
Hal-CH₂-B' (VIII)
wherein Hal represents a halogen atom and B' represents a linear or branched (C₁-C₅)alkyl group or linear or branched (C₂-C₅)alkenyl group, those groups being substituted by an R'⁵ group, by a group of formula (II') 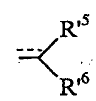 or by a group of formula (III') 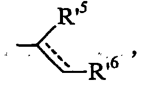 in which groups R'⁵ an d R'⁶ and the representation ---- are as defined hereinbefore,
to yield a compound of formula (I/c), a particular case of the compounds of formula (I): 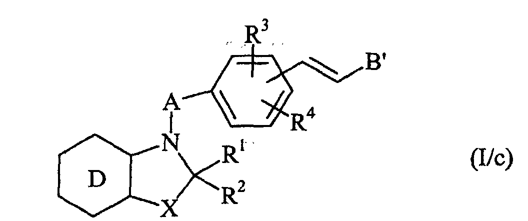 wherein D, X, A, B', R¹, R², R³ and R⁴ are as defined hereinbefore,
which may be subjected to catalytic hydrogenation, in the presence of palladium for example, to obtain a compound of formula (I/d), a particular case of the compounds of formula (I): 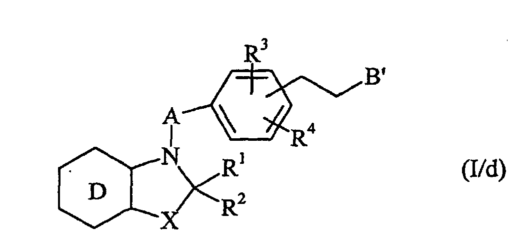 wherein D, A, X, B', R¹, R², R³ and R⁴ are as defined hereinbefore,
- or which compound of formula (VI) is converted into a corresponding acid chloride which is condensed, in the presence of a palladium or tin compound for example, with a compound of formula (IX): 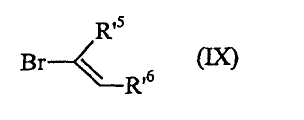
wherein R'⁵ and R'⁶ are as defined hereinbefore,
to yield a compound of formula (X): 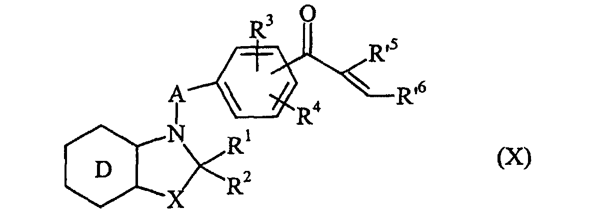 wherein D, X, A, R¹, R², R³, R⁴, R'⁵ and R'⁶ are as defined hereinbefore,
which is subjected to the action of a reducing agent, such as Et₃SiH for example, to yield a compound of formula (I/e), a particular case of the compounds of formula (I) : 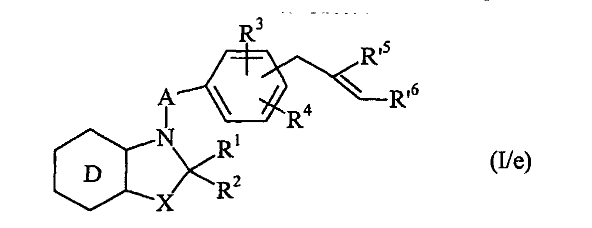 wherein D, X, A, R¹, R², R³, R⁴, R'⁵ and R^{'6} are as defined hereinbefore,
wherein the compounds of formula (I/a), (Vb), (I/c), (I/d) or (I/e) in which R'⁵ and R'⁶ represent ester groups may be fully or partially hydrolysed to yield corresponding gemdicarboxylated or hemicarboxylated compounds of formula (I/f), a particular case of the compounds of formula (I): 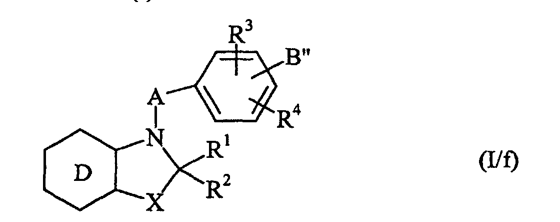 wherein D, X, A, R¹, R², R³ and R⁴ are as defined hereinbefore and B" represents a group as defined hereinbefore for B in which R⁵ and/or R⁶ represent(s) a group COOH,
(it being possible for the compounds of formula (I/f) wherein B" represents a group - CH=CH-COOH to be obtained directly from a compound of formula (VI) by the condensation of malonic acid under decarboxylating conditions and for them to be reduced to yield compounds of formula (I/f) wherein B" represents a group -CH₂-CH₂-COOH),
the compounds of formulae (I/a) to (I/f) constituting the totality of the compounds of the invention, which compounds may be purified according to a conventional separation technique, are converted, if desired, into addition salts with a pharmaceutically acceptable acid or base, and are optionally separated into their isomers according to a conventional separation technique.

24. Pharmaceutical compositions comprising as active ingredient at least one compound of formula (I) according to any one of claims 1 to 22 or a pharmaceutically acceptable addition salt thereof with an acid or a base, alone or in combination with one or more pharmaceutically acceptable excipients.

25. Pharmaceutical compositions according to claim 24 for use in the preparation of a medicament for the treatment and/or prophylaxis of hyperglycaemia, dyslipidaemia and, more especially, in the treatment of non-insulin dependent type II diabetes, insulin resistance, glucose intolerance, disorders associated with syndrome X, coronary artery disease and other cardiovascular diseases, renal disorders, retinopathy, disorders associated with the activation of endothelial cells, psoriasis, polycystic ovary syndrome, dementia, osteoporosis, intestinal inflammatory disorders, myotonic dystrophy, pancreatitis, arteriosclerosis, xanthoma, but also in the treatment or prevention of type I diabetes, obesity, regulation of appetite, anorexia, bulimia, anorexia nervosa, as well as cancer pathologies and especially hormone-dependent cancers, such as breast cancer or colon cancer, and as angiogenesis inhibitors.
